(19) Europäisches Patentamt
European Patent Office
Office européen des brevets

(11) EP 4 252 749 A1

(12) EUROPEAN PATENT APPLICATION
published in accordance with Art. 153(4) EPC

(43) Date of publication:
**04.10.2023 Bulletin 2023/40**

(21) Application number: **21848149.7**

(22) Date of filing: **18.11.2021**

(51) International Patent Classification (IPC):
***A61K 31/167*** *(2006.01)* ***A61K 31/27*** *(2006.01)*
***A61P 25/28*** *(2006.01)* ***A61K 9/00*** *(2006.01)*
***A61K 9/20*** *(2006.01)* ***A61K 47/06*** *(2006.01)*

(52) Cooperative Patent Classification (CPC):
**A61K 31/167; A61K 9/0043; A61K 9/2027; A61K 9/2054; A61K 31/27; A61K 47/10; A61K 47/22; A61K 47/26; A61P 25/28**

(86) International application number:
**PCT/CU2021/050012**

(87) International publication number:
**WO 2022/111742 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.11.2020 CU 20200087**

(71) Applicant: **Centro De Neurociencias De Cuba
Playa La Habana 11600 (CU)**

(72) Inventors:
- **RODRÍGUEZ-TANTY, Chryslaine
11300 La Habana (CU)**
- **SABLÓN CARRAZANA, Marquiza
17100 La Habana (CU)**
- **MENÉNDEZ SOTO DEL VALLE, Roberto
10400 La Habana (CU)**
- **BENCOMO MARTÍNEZ, Alberto
32300 Artemisa (CU)**
- **RIVERA MARRERO, Suchitil
10800 La Habana (CU)**
- **GARCÍA PUPO, Laura
17100 La Habana (CU)**
- **GONZÁLEZ MESA, Leonora
11500 La Habana (CU)**
- **LEÓN CHAVIANO, Samila
17100 La Habana (CU)**
- **ÁGUILA CORDOVA, Adriana
10400 La Habana (CU)**
- **CASTRO-PALOMINO ANTELA, Kathleen
10400 La Habana (CU)**
- **PENTÓN ROL, Giselle
11300 La Habana (CO)**
- **OTAÑO TAMAYO, Laura
10800 La Habana (CU)**
- **PÉREZ PERERA, Rafaela
17100 La Habana (CU)**
- **CERVANTES LLANOS, Majel
11300 La Habana (CU)**
- **DÍAZ GARCÍA, Orestes de Jesús
17100 La Habana (CU)**
- **DORESTE BROWN, Miriam
11300 La Habana (CU)**

(74) Representative: **V.O.
P.O. Box 87930
2508 DH Den Haag (NL)**

(54) **PHARMACEUTICAL COMPOSITION OF NAPHTHALENE DERIVATIVES AS MULTI-TARGET THERAPEUTIC AGENTS FOR THE TREATMENT OF ALZHEIMER'S DISEASE**

(57) This invention relates to Pharmaceutical Chemistry and concerns the pharmaceutical composition of compounds, which show multi-target action on the cholinergic, glutamatergic and mitochondrial systems that are affected in Alzheimer's Disease (AD), whose general Formula I is,

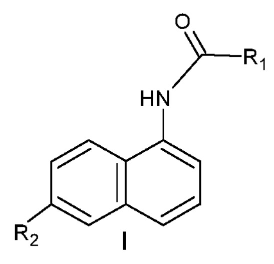

I

wherein the substituents R1 and R2 are set forth in the description and claims.

The formulation of these compounds increases efficacy and tolerance through oral, sublingual, parenteral, transdermal and nasal administration. They can be used on their own, as monotherapy, replacing the multitherapy currently used for AD.

The formulation of these compounds, their salts, hydrates, enantiomers, isomers, metabolites, prodrugs for administration to humans, as active ingredients for the treatment of AD, increases bioavailability, residence time of the active ingredient, and adequate excretion, which increases efficacy, biosafety, adherence and tolerance to treatment.

## Description

The present invention is related to Pharmaceutical Chemistry and refers to the pharmaceutical composition of the compounds of Formula I, which show multi-target action on the different mechanisms of the cholinergic, glutamatergic and mitochondrial systems that are affected by Alzheimer's disease (AD). The formulation of these compounds increases efficacy and tolerance through their oral, sublingual, parenteral, transdermal and nasal administration.

This invention is particularly useful to provide an effective formulation, in which the compounds of Formula I, their salts, hydrates, enantiomers, isomers, metabolites, and prodrugs for administration to humans, are considered as active substances or principles for the treatment of AD.

With the increase in life expectancy of the world population, there has been an increase in neurodegenerative diseases, mainly those related to senile dementias. In particular, Alzheimer's disease (AD) has a high incidence in the population over 60 years of age (between 50% - 60%). This disease leads to progressive dementia, characterized by the occurrence of amnesic episodes, disorientation, executive and cognitive function disorders, hallucinations, depression and agitation.

At present, the exact mechanism that produces this disease of complex etiology is still under discussion. According to experimental evidence, the hypothesis of the amyloid cascade and that of the phosphorylation of the tau protein play a fundamental role in its origin (Hardy JA, et al. in Science, 1992; 256: 184-5; Haass C. et al. in Cold Spring Harb Perspect Med. 2012; 2: a006270; Mucke L et al. in Cold Spring Harb Perspect Med. 2012; 2: a006338). However, there are also other alternative hypotheses supported by scientific findings, such as the alteration of mitochondrial activity, the neuroinflammation hypothesis and the hypothesis on the role of metabolism (related to cholesterol and insulin) and the dendritic hypothesis. (Castello MA et al. in Aging Res Rev. 2013; 12: 282-8; Castello MA et al. in Aging Res Rev. 2014; 13: 10-2; Drachman DA. in Alzheimers Dement 2014; 10: 372-80; Ferreira ST et al. in Alzheimers Dement 2014; 10 (1 Suppl): S76-83; De Felice FG, et al. in Diabetes 2014; 63: 2262-72; De Felice FG in J Clin Invest. 2013; 123: 531-9; Cochran JN et al. in Brain Res Bull. 2014; 103: 18-28).

In general, the neuropathology of AD manifests itself as an inflammatory immune reaction, which involves the activation in the brain of both the innate and adaptive immune systems, which leads to a neurochemical cascade that involves the aggregation of β-Amyloid peptides (βA) and tau protein. It is suggested that the presence of amyloid fibers or plaques sequentially generates the complement activation, microglia, the release of cytokines (chemokines), and finally, diffuse neurotoxicity (Reitz C. in International Journal of Alzheimer's disease. 2012 Jan; 2012: 369808; Rodrigue K. in Neuropsychol Rev. 2009 December; 19 (4): 436-450. Doi: 10.1007 / s 11065-009-9118-x; Bateman, RJ et al. In Alzheimer's disease. N. Engl. J Med. 367, 795-804, 2012; Karran, E., et al. in Nat. Rev. Drug Discov. 10, 698-712, 2011). On the other hand, Soyon Hong et al. in Science (2016, doi: 10.1126 / science.aad8373), describe that in the initial stage of asymptomatic AD there is an early synaptic loss mediated by complement and microglia, specifically by an increase in the C1q protein, before the deposition of βA plaques. Also, it is known that the participation of the βA peptide in its initial state of aggregation is crucial for the overexpression of C1q. Chronic inflammation as an immune response in the brain plays an important role in progressive neuronal death in many degenerative diseases. Thus, the phagocytic system, made up of infiltrated macrophages and monocytes attached to resident microglial cells, produces excessive amounts of cytokines such as tumor necrosis factor α (TNF α), interleukin 1 (IL1), as well as reactive oxygen species (EROS) and nitrogen (ENSO), which are involved in AD. This set of responses to given stimuli triggers a vicious cycle of aggressions that is sustained by localized glial cells, which provoke a phagocytic attack that irreversibly damages neurons (Malm TM, in Neurotherapeutics 2015, 12: 81-93; ElAli A. et al. in Brain, Behavior, and Immunity, 2015, doi: 10.1016 / j.bbi.2015.07.021; Michaud J.-P. et al., in Neuron, 85, 4, 450-2, 2015) .

Mitochondrial dysfunctions are described as an early event in the pathophysiology of AD and appear before βA deposition and memory deficit in AD patients and transgenic mice (Maurer, I., et al. in Neurobiol. Aging 2000, 21, 455-462; Caspersen, C. et al. in FASEB J. 2005, 19, 2040-2041; Mosconi, L et al. in Ann. NY Acad. Sci. 2008, 1147, 180-195). During AD pathogenesis, βA oligomers accumulate in mitochondria, leading to impaired energy metabolism, increased oxidative stress (OE), and apoptosis (Lustbader, et al. in Science 2004, 304, 448 -0452; Caspersen, C. et al. In FASEB J. 2005, 19, 2040-2041; Manczak, et al. in Hum. Mol. Genet. 2006, 15, 1437-1449). In studies carried out in AD patients and transgenic mice, the constant decreases in the enzymes of the tricarboxylic acid cycle and in the activities of cytochrome c oxidase are described (Yates, et al. in J. Neurochem 1990, 55, 1624-1630 ; Maurer, I., et al. in Neurobiol Aging 2000, 21, 455-462; Caspersen, C. et al. in FASEB J. 2005, 19, 2040-2041; Leuner, et al. in Mol. Neurobiol. 2012, 46, 186-193). Something similar is described for the mitochondria isolated from the brains of transgenic mice and in the isolated mitochondria exposed to βA oligomers in which an increase in EO has also been found, a key element in the physiology of AD (Casley, et al., in Neurobiol. Dis. 2002a. 10, 258-267; Aleardi, AM, et al., in J. Bioenerg. Biomembr. 2005, 37, 207-225; Caspersen, C. et al. in FASEB J. 2005, 19, 2040-2041; Clementi, ME et al. in FEBS Lett. 2005, 579, 2913-2918; Leuner, et al. in Mol. Neurobiol. 2012, 46, 186-193; Smith, MA, et al. in Nature 1996, 382, 120-121; Leuner, et al. in Mol. Neurobiol. 2012, 46, 186-193).

A possible mechanism of mitochondrial dysfunction is related to the opening of the mitochondrial permeability

transition pore (mPTP), which can be triggered by $Ca^{2+}$ and EO overload (Kroemer, G. and Reed, JC in Nat. Med. 2000, 6, 513-519) and that induces the release of pro-apoptotic factors, such as the proapotic protein Bax that contributes to the formation of mPTP (Jürgensmeier, JM, et al. in Proc. Natl. Acad. Sci. USA, 1998, 95, 4997-5002; March, I., et al. in Science 1998, 281, 2027-2031; Narita, M., et al. in Proc. Natl. Acad. Sci. USA 1998, 95, 14681-14686). Consequently, cytochrome c, which promotes apoptosis, is released by binding to the apoptosis protease activating factor (APAf-1) and forming a complex known as "apoptosome" (Liu, X., et al. in Cell 1996, 86, 147-157; Yang, J., et al. in Science 1997, 275, 1129-1132).

Furthermore, it is known that mitochondrial bioenergetic dysfunction associated with βA promotes glutamatergic excitotoxicity and therefore cell death. By reducing cellular energy stores, the cytoplasmic membrane potential is affected, which eliminates the $Mg^{2+}$blockade of N-methyl-D-aspartate (NMDA) receptors. This allows persistent activation by endogenous glutamate, and supposes an increase in the entry of $Ca^{2+}$ ions into the cell, which causes excitoxicity in neurons, an event that has been related to memory problems characteristic of AD (Zádori D. in Journal of Alzheimer's Disease, 2018, 62, 523-547). Supporting this hypothesis is the demonstrated fact that glutamate antagonists reduce mitochondrial toxin-induced neuronal death. These and other subsequent evidences suggest that ROS generated by the electronic transport chain and / or by intracellular pathways linked to the activation of the glutamate receptor, could be the cause of the degeneration of neurons (Villegas S. in Medicina Clinica, 2015, 145, 2, 76-83).

The neuropathological complexity of AD indicates that it is a multifactorial disease, however, from the therapeutic point of view, it has not been approached that way up to now.

There are only four drugs approved by regulatory entities. These belong to two groups: acetylcholinesterase inhibitors (AChEI) and N-methyl-D-aspartic acid receptor antagonists (NMDAr) (Chiang K et al. in Annu Rev Pharmacol Toxicol. 2014; 54: 381- 405; Francis PT et al. in Trends Pharmacol Sci. 2005; 26: 104-11; Huang Y. et al. In Cell. 2012; 148: 1204-22.)

The AChEIs are donepezil, rivastigmine, and galantamine. Their action mechanism is to increase cholinergic transmission by inhibiting acetylcholinesterase in the synaptic cleft and, therefore, they could increase the cognitive capacity of patients with AD. AChEIs are available in various formulations that include immediate release forms, such as tablets, capsules and solutions, as well as the controlled release form for oral administration, among others (Villegas S. in Medicina Clinica, 2015, 145, 2, 76-83). However, these AChEIs offer only moderate clinical benefit for some patients, even when these drugs are administered at their maximum doses for safety and tolerability. Additionally, these compounds cause side effects including anorexia, nausea, vomiting, diarrhea, abdominal pain, and weight loss (in Physicians Desk Reference 2008, Thomson PDR et al. in Clinical Geriatrics: Volume 2011, 19, 1; Ali T. et al. in PLOS ONE, 2015, 7, 1-10; Tsoi KKF in JAMDA, 1-7, 2015, doi: 10.1016 / j.jamda.2015.08.007).

On the other hand, NMDAr are ionotropic receptors for glutamate, a neurotransmitter that acts as an essential component in neuronal synaptic plasticity and memory. Memantine, an antagonist of these receptors, is used in clinical practice for its neuroprotective effect indicated in moderate to severe AD *(National Institute for Clinical Excellence. Technology appraisal guidance 217. Donepezil, galantamine, rivastigmine and memantine for the treatment of Alzheimer's disease at www.nice.org.uk/guidance/TA217;* O'Brien JT, et al. in J Psychopharmacol Oxf Engl. 2011; 25: 997-1019*;* Francis PT et al. in Trends Pharmacol Sci. 2005; 26: 104-11*;* Huang Y et al. in Cell. 2012; 148: 1204-22). In order to reduce the side effects of memantine, a controlled release formulation has been developed that allows the release of the drug in two time stages (in US 5,382,601), and / or the administration of a single daily dose (in US 2006/0051416). However, this treatment also does not stop the progression of the disease, it only has beneficial effects during some stages and they vary greatly depending on the patient.

Currently, there is no effective treatment for this disease, which justifies the development of other therapeutic strategies aimed at finding other targets of this complex etiology, such as:

- ACh receptor antagonists (Home-ClinicalTrials.gov [accessed March 21, 2016] Available at: http: //www.cli-nicaltrials.gov/. Zawieja P, et al. in Geriatr Gerontol. Int. 2012; 12: 365 -71 Frolich L, et al. in J Alzheimers Dis. 2011; 24: 363-74.
- Inhibitors of βA aggregation (Aisen PS, et al. In Arch Med Sci. 2011; 7: 102-11. Caltagirone C, et al. in Alzheimers Dis. 2010; 20: 509-16. Salloway S, et al in Neurology. 2011; 77: 1253-62. Home-ClinicalTrials.gov [accessed March 21, 2016]. available at: http: //www.clinicaltrials.gov/)
- Protease inhibitor / activator (Yan R, et al ... in Lancet Neurol. 2014; 13: 319-29. Vellas B, et al. in Curr Alzheimer Res. 2011; 8: 203-12. Xia W, and co. in J Alzheimers Dis. 2012; 31: 685-96.)
- Lipid-lowering drugs such as statins (Home-ClinicalTrials.gov [accessed March 21, 2016]. Available at: http: //www.cli-nicaltrials.gov/. Wong WB. et al. in Pharmacoepidemiol Drug Saf. 2013; 22: 345 -58; Frolich L, et al. in J Alzheimers Dis. 2011; 24: 363-74.)
- Active Immunotherapy (Panza F. et al. in Expert Rev Clin Immunol. 2014; 10: 405-19; Ryan R. JM et al. in J Alzheimers Dis. 2009; 17: 243; Winblad B. et al. in Lancet Neurol. 2012; 11: 597-604; Schneeberger A. et al. in N Engl J Med. 2014; 370: 322-33.)

- Passive Immunotherapy (Home-ClinicalTrials.gov [accessed March 21, 2016] Available at: http: //www.cli- nicaltrials.gov/. Panza F. et al. in Expert Rev Clin Immunol. 2014; 10: 405- 19; Salloway S. et al. in N Engl J Med. 2014; 370: 322-33; Doody RS. et al. in N. Engl J Med. 2014; 370: 311-21; Ostrowitzki S. et al. in Arch Neurol. 2012; 69: 198-207).

None of these therapeutic strategies have been approved for use in humans, nor have they proven their efficacy to date, and even the vast majority have negative or contradictory results. Neither in the development of these strategies, was the concept of developing a compound capable of interacting positively in different targets affected in the cerebral metabolism of subjects suffering from AD.

Carrazana et al. (in WO2010118706 A4) declare the synthesis of new neutral, lipophilic and low molecular weight compounds that cross the blood-brain barrier (BBB) and adhere to amyloid plaques, and that are used as diagnostic agents, through SPECT, PET and MRI techniques, for the visualization of these structures. In this invention it is stated that these compounds can be used as therapeutic agents for Alzheimer's and Parkinson's diseases. On the other hand, these authors state in WO2014131374 A1, the use of these compounds as therapeutic agents for this disease because they can: inhibit, reduce, refold, and disaggregate soluble oligomers, prefibrillar structures, protofibrils and amyloidogenic fibers, as well as amyloid plaques. However, these patents do not declare the anticholinergic, antioxidant, antiglutamatergic and anti-inflammatory action of these compounds, nor their finding and conception as a multi-target target therapeutic agent, which allows the development of a novel monotherapy, which would overcome the difficulties that until now exist in the treatment of AD. Neither is it declared an effective formulation for the administration of the compounds of Formula I, their salts, hydrates, enantiomers, isomers, metabolites, prodrugs for their administration to humans, as active substances or principles for the treatment of AD.

Currently, the development of controlled release pharmaceutical forms is practically associated with the use of polymeric materials with very precise specifications in the biopharmaceutical field. Thanks to this, they are able to maintain the drug concentration on the therapeutic plateau using a single dose, as well as to release it continuously in a given time (Rodriguez, IC; Cerezo, A.; Salem, in II Bioadhesive delivery systems. Ars Pharmaceutica, 2000, 41, 1, 115-128; Vintiloiu, A., Leroux, JC in Organogels and their use in drug delivery. Journal of Controlled Release, 2008, 125, 179-192). In matrix systems, the active principle is uniformly distributed within a polymer, either as a suspension or as a solution, and its release kinetics will depend on the structure of the matrix and the chemical nature of the materials used (Frenkel, J., in Rubber Chemistry and Technology, 1940, 13, 264-274; Tanaka et al. In Encyclopedia of Polymer Science, 1986, 2nd ed. 6, 514; Fyfe, CA, Blazer, AI, in Journal of Controlled Release, 1998, 52, 221-225). The delayed or modified acting excipients that are used to formulate matrix tablets can be insoluble or inert polymer matrices, lipid or water insoluble matrices or hydrophilic matrices.

Hydrophilic matrices are obtained by mixing the active principle with hydrophilic polymers, which gives rise to polymeric matrices. Cellulose ethers are hydrophilic polymers widely used in the development of controlled release systems, being biologically compatible and non-toxic. Among the best known we have: hydroxypropylmethylcellulose (HPMC), hydroxypropylcellulose (HPC), sodium carboxymethylcellulose (CMC Na), methylcellulose (MC), ethylcellulose (EC) and microcrystalline cellulose polysaccharides and their derivatives, polyethylene glycine oxides, polyethylene glycols chitosan, poly (vinyl alcohol), xanthan gum, maleic anhydride copolymers, poly (vinyl pyrrolidone), starch and starch-based polymers, maltodextrins, poly (2-ethyl-2-oxazoline), poly (ethyleneimine), hydrogels polyurethane, cross-linked polyacrylic acids and their derivatives. In the formulations in which these matrices are used, with other suitable excipients, it is possible to prepare tablets, or in rigid gelatin capsules (Dabbagh, MA, et al. In International. Journal of Pharmaceutics, 1996, 140, 85-95)

Recently, much interest has been focused on exploring the intranasal route for drug delivery to the brain due to the high permeability of the nasal epithelium, which allows the administration of drugs of higher molecular mass, and a rapid rate of drug absorption with plasma concentration profiles sometimes nearly identical to intravenous injections (Michael IU, et al. in J Pharm Pharmacol 2001; 53: 3-22). Other advantages of this route are: large surface area for the absorption of drugs, better adherence to treatment by the patient, rapid obtaining of therapeutic blood levels, does not present aggressive conditions and avoids the effect of the first hepatic pass.

In the literature, it is described that the uptake of the drug in the brain from the nasal mucosa occurs mainly through three different pathways (Ilium L. in Eur J Pharm Sci 2000; 11: 1-18; Frey WH II. in Drug Deliv Tech 2002; 2: 46-49. Vyas TK, et al. in Curr Drug Deliv 2005; 2 (2): 164-175). One is the systemic pathway where the drug is absorbed by the systemic circulation and subsequently reaches the brain by crossing the BBB. The other two direct pathways are the olfactory pathway and the trigeminal-neural pathway, by which the drug travels in part from the nasal cavity to the cerebrospinal fluid (CSF) and brain tissue (Thorne RG, et al. in Neuroscience 2004; 127: 481-496). To achieve drug delivery to the CNS, drugs must penetrate efficiently and rapidly through the nasal mucosa. Thus, the design of the dosage form plays a critical role in pharmacokinetics and bioavailability after intranasal administration. The retention of the drug in the place of absorption and the intimate contact are two important factors to take into account in the formulation. It is known that as the viscosity of the formulation increases, the residence time of the formulation in the nasal cavity

increases and, therefore, the probability of absorption (Pires, A, et al. In J Pharm. Pharmaceut Sci. 2009; 12 (3): 288-311). On the other hand, the nasal mucosa and the mucus layer contain a wide variety of enzymes, for which several techniques have been tried to prevent enzymatic degradation, including the use of protease and peptidase inhibitors (Pires, A , et al. in J Pharm. Pharmaceut Sci. 2009; 12 (3): 288-311). Also, there are absorption promoters that produce reversible modifications of the epithelial barrier, increase the permeability of this barrier by modifying the lipid membrane, increase its fluidity, or open the intercellular spaces, which increases paracellular transport. These compounds are related to an increase in tissue damage, however, some compounds such as chitosan, cyclodextrins and some phospholipid derivatives, present an increase in absorption and bioavailability, which outweigh any negative effect derived from the modification of the mucosa (Pires, A. et al. in J Pharm. Pharmaceut Sci. 2009; 12 (3): 288-311; Casettari, L. and Illum, L. in J. Control. Release 2014, 190, 189-200).

Mucoadhesive compounds allow the binding of the pharmaceutical form to the mucosa layer, which increases the contact time between the mucosa and the drug, and favors absorption. These include agarose, chitosan, gelatin, carrageenan, pectin, cellulose derivatives, polyacrylates, PEG, methacrylate, acrylic acid, PVA aminodextran, chitosan-EDTA, PAC, alginate, carboxymethylcellulose and its sodium form (CMC and Na-CMC), hydroxyethylated starch, polyvinylpyrrolidone (PVP), thiolated polymers, polyacrylic acid, (Mansuri, S. et al. in React. Funct. Polym. 100 (2016) 481151 - 172).

The intranasal route can be used for the transport of drugs of low molecular mass (less than 1000 Da) (Behl CR, et al. in Adv Drug Del Rev 1998; 29 (1): 89-116). Various patents declare different approaches to the delivery of these small molecules to the CNS.

Quay, S.C. et al. (in US20030225031A1 (2003) and in US20060003989A1 (2006)) claim pharmaceutical compositions for the treatment of dementia, AD, learning disorders, nicotine withdrawal syndrome by acetylcholinesterase inhibitors (donepezil, tacrine, rivastigmine, galantamine, among others), which comprises a liquid solution, gel or powder for the nasal administration of the drug and a permeability enhancer for the transmucosal absorption of the drug in the CSF by intranasal administration.

Went G.T. et al in US20050245617A1 (2005) claim a pharmaceutical composition of an N-methyl-D-aspartate (NMDA) receptor antagonist (memantine), a monoamine oxidase (MAO) inhibitor or a GADPH (selegiline) inhibitor; and a pharmaceutical acceptable carrier, the composition of which is administered as a sustained release dosage form for oral, topical transepithelial, subdermal, intravenous, intranasal, or inhalation administration for the treatment of CNS-related conditions, such as Parkinson's disease, multiple sclerosis and AD. These authors in US20060252788A1 (2006) declare a composition where they combine memantine with donepezil, and in US20060189694A1 (2006) they reveal a composition of a derivative of aminoadamantine (memantine, rimantadine, amantadine) and of a decarboxylase inhibitor (Levodopa, Carbidopa) or a catechol-O-methyltransferase inhibitor (talcapone, entacapone), together with carriers in an extended-release dosage form for the treatment of various neurodegenerative conditions.

Cummings, C.J., et al. in US20070037800A1 (2007) declare an intranasal method to treat neurological disorders such as Huntington's disease or AD through the use of clotrimazole and its derivatives. On the other hand, Tao, T. et al. in CN1621039 (2005) disclose the bioavailable preparation of Huperzine-A and its derivative or salt, which is administered nasally for the prevention and treatment of senile dementia and the improvement of memory and learning capacity of adolescents.

The present invention is related to Pharmaceutical Chemistry and refers to the pharmaceutical composition of the compounds of Formula I, which show multi-target target action on the cholinergic, glutamatergic and mitochondrial systems that are affected in Alzheimer's disease (AD). The formulation of these compounds increases efficacy and tolerance through their oral, sublingual, parenteral, transdermal and nasal administration.

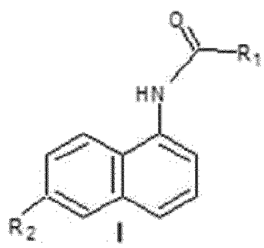

I

Where:

$R_1$: -alkylenyl-C (O) NH-alkylenyl-$R_3$, -alkylenyl-C (O) O-$R_4$;

$R_3$: -COOH, -OH, -SH, -$NH_2$, -NH-alkyl-, -NH-alkylenyl-$NH_2$, -NH-alkylenyl-NH-C (O) -alkylenyl-S-$R_5$, -NH-dithiocarbamate- alkyl, -N-alkyl-dithiocarbamate alkaline earth metal salts; or salts of the groups mentioned above, phar-

maceutically acceptable.

$R_4$: succinimidyl group;

$R_5$: -H, -C (O) -alkyl, -C (O) -$C_6H_5$; and

$R_2$: -H, -alkyl.

The term "alkyl" is characterized by being a linear or branched aliphatic chain of saturated carbon atoms and hydrogen atoms, preferably methyl or ethyl. The term "alkylenyl" refers to a divalent analog of a linear or branched alkyl group, preferably methyleneyl (-$CH_2$-), ethylenyl (-$CH_2CH_2$-), or propylenyl (-$CH_2CH_2CH_2$-).

Specifically, the formulated compounds of Formula I can be used alone as a substitute for the multi-therapy that is used today, where a combination of several active principles is used to improve the symptoms of AD. The compounds of Formula I individually present anticholinergic, antioxidant, antiglutamatergic and anti-inflammatory activity, which makes them potent active principles for the monotherapy of AD. Furthermore, they are capable of inhibiting amyloidogenic peptide aggregation and plaque formation (in WO2010118706 A4*).* With this, the patient receives a monotherapy, which significantly improvement the clinical symptoms and the biological alterations present in the brain of the subjects suffering from AD.

The formulation presented of the compounds of Formula I, their salts, hydrates, enantiomers, isomers, metabolites, prodrugs for their administration to humans, as active substances or principles for the treatment of AD, allows to increase the bioavailability, the time of residence of the active principle, and adequate excretion which increases the efficacy, biosafety, adherence and tolerance to treatment, through oral, sublingual, parenteral, transdermal and nasal administration.

The compounds of Formula I can be conveniently prepared as described in Cuban patent No. 2009-57, PCT-CU2010-000001.

In the present invention, the compounds of Formula I unexpectedly exhibit mito-protective properties. Figure 2 (from non-limiting Example 1) shows the effect of compound **1** on the dissipation of the mitochondrial membrane potential ($\Delta\psi$), related to the flow of electrons through the protein complexes of the electron transport chain (CTE). Mitochondria treated with ethylene-bis (oxyethyleneitrile) tetraacetic acid (EGTA) show the least dissipation of mitochondrial potential, as this is the control group of the experiment. In this case, EGTA acts as a chelator of the $Ca^{2+}$ pollutant in the reaction medium, so that the mitochondria are in their best state and retain their potential to the maximum. The EGTA + 3-chlorophenyldrazone carbonyl cyanide (CCCP) control is the group where mitochondrial damage occurs, because the CCCP dissipates $\Delta\psi$ by acting as an oxidative phosphorylation uncoupler. Compound **1** in the concentration range from 0.1 $\mu$mol/L to 100 $\mu$mol/L shows similar potential dissipation values to the control group (EGTA). According to these results, compound 1, in the dose range tested, decreases the susceptibility to dissipation of the potential.

Figure 3 shows the results of the evaluation of mitochondrial swelling induced by 50 $\mu$mol/L of $Ca^{2+}$ and 2 mmol/L of inorganic phosphate (Pi), which was evaluated spectrophotometrically from the decrease in absorbance at 540 nm. In this case, it was observed that in the control group (in the presence of $Ca^{2+}$) the maximum absorbance values are shown, as a measure of maximum swelling. On the other hand, the samples treated with EGTA (chelator of $Ca^{2+}$) showed low absorbance values ($p < 0.001$), as this was the undamaged control. The group treated with CCCP also shows low absorbance values ($p < 0.001$), since when uncoupling it prevents the entry of $Ca^{2+}$ ions, and thus prevents mitochondrial swelling. Compound **1** at similar concentrations reverses the effect of $Ca^{2+}$-induced swelling, so it can be considered as a protector of mitochondrial integrity and functionality. These effects correspond to the observed effects on the dissipation of the mitochondria membrane potential.

The effect of compound **1** on the production of hydrogen peroxide ($H_2O_2$) was also evaluated, which is directly related to the state of preservation of the mitochondrial membrane potential ($\Delta\psi$). Under basal conditions, the mitochondria produce high amounts of reactive species, as corroborated in the control group treated with EGTA. When the treatment with CCCP was carried out, a similar behavior was observed (Figure 4, from non-limiting Example 1), but in this case it is deduced that the generation of reactive species is associated with the uncoupling that induces CCCP, and not with the production of ATP by the mitochondrial ATPase, that is to say to an optimal state of functionality of the mitochondria. Treatment with compound **1** protects against $H_2O_2$ production in the dose range tested.

In summary, our results suggest that this compound acts as a protective agent of mitochondrial function since the mitochondria were less susceptible to the dissipation of the membrane potential, it reduces swelling and the generation of reactive oxygen species.

Based on *in silico* studies, compounds of Formula **I** interact with the enzyme AChE in a similar way as donepezil. These interactions are hydrophobic with the amino acids Trp 84, Trp 279 and Phe 330. In the same way, these compounds interact with all the serine residues in the cavity involved in the reaction with acetylcholine, by forming three hydrogen bonds that give stability to the ligand / enzyme bond, suggesting that the compounds may block enzyme activity. The

Scopolamine-induced amnesia (ESC) model is one of the most widely used to induce cognitive deficits associated with dementia and aging (Bajo R, et al. in Scientific reports 2015, 5: 9748*;* Gilles C and Ertle S. in Dialogues Clin. Neurosci. 2000, 2 (3): 247-255*;* Haider S et al. in Brain Res. Bull. 2016, 127 (Supplement C): 234-247*)*. The use of this competitive non-selective muscarinic cholinergic receptor antagonist is an effective way to block cholinergic neurotransmission and thus induce dementia. Also, ESC induces an increase in the activity of the enzyme acetylcholine esterase AChE, which contributes together with the blocking of the receptors to the deficit of cholinergic neurotransmission. This model is used in in vivo tests of those inhibitors of enzyme activity such as donepezil (Shin CY, et al. in Biomolecules & Therapeutics 2018, 26 (3): 274-281). Along with these effects, other mechanisms such as EO induction, neuroinflammation, mitochondrial dysfunction, decreased phosphorylation of cAMP and negative regulation of NFDB (neurotropic factor derived from the brain) are observed. and the glia acidic fibrillar protein (GFAD) (Haider et al., in Brain Research Bulletin 2016, 127, 234-247*;* Jung et al., in Biol. Pharm. Bull. 2009, 32 (2), 242 -246*;* Konar et al., in PLOS ONE, 2011, 6, 11, e27265*;* Lee et al., in Scientific reports, 2015, 5, 9651 *.;* Wong-Guerra et al., in Neurol Res. 2017; 39 (7), 649-659*),* all of which contribute to the cognitive dysfunction observed in this model.

Behavioral assessment in the acute ESC model was performed using the "spatial version" of the Y-maze, which assesses short-term spatial memory *(*McGaugh JL, in Science, 153 (1966) 1351*),* and is based on the ability to the animals in spontaneously recognizing new spaces within the maze. The memory evaluated in this protocol involves the function of the hippocampus (Csiszar A., et al. in Am. J. Physiol. Heart Circ. Physiol. (2013), 305 H1120-1130*;* Olton DS and Paras BC, in Neuropsychologia, 17 (1979) 669-682.*)*

Figure 5 (Non-limiting example 2) shows the results of the behavioral test in the Y-maze of the compound methyl (2 - {[4- (1-naphthylamino) -4-oxobutanoyl] amino} ethyl) dithio carbamate (**3**), evaluated in the ESC model. In this experiment, it was observed that the control group animals have a preference for the novel arm, which was evidenced by a significant decrease in the time spent in the two family arms (p <0.001). This preferential behavioral profile was not reproduced with the administration of ESC 30 minutes before training. In this case, the time spent in the novel arm decreased, with no statistically significant differences being observed with respect to the other two arms. In contrast, in the group where compound 3 was administered (doses from 0.1 mg/kg to 10 mg/kg), the amnesia induced by ESC was significantly reversed (p <0.001). Thus, the animals exhibited a preferential behavioral profile analogous to that of the control group, characterized by a significant increase in permanence in the novel arm compared to the familiar arms. In this trial, donepezil (positive control; dose: 1 mg/kg, ip) administered 30 minutes before the test, also reversed the deficit induced by ESC, which is in agreement with other authors and with the anticholinergic effects of this drug (Lee JS, et al. in Scientific reports 2015, 5: 9651*;* Riedel G, et al. in Behav. Brain Res. 2009, 204 (1): 217-225*).* Thus, our results indicate that compound **3**, administered in single doses in the range of 0.1 to 10 mg/kg, 30 minutes before training, surprisingly prevents the amnestic effect induced by the administration of ESC. The residence times in the novel arm of the different experimental groups were compared (Figure 6, Non-limiting Example 2). Thus, the time of the control group vs that of ESC showed a clear decrease in the latter group (control: 133.8 $\pm$ 9.3 sec; ESC: 98.8 $\pm$ 4.2 sec), which was statistically significant (p <0.001). The behavior of the animals in the groups treated with compound **3** depended on the concentration administered. Up to the 1 mg/kg dose there was a gradual increase in the exploration time, significantly different from the ESC group (p <0.01). At higher doses, a decrease in this parameter was observed. In the case of the group treated with donepezil, as expected, the residence time was significantly longer than that of ESC. In summary, in this experience the dose of **3** of 1 mg/kg behaved as the dose with the greatest effect.

For the study of long-term memory (McGaugh JL, in Science, 153 (1966) 1351) the "water maze" has been developed (called Morris's Water Maze, MWM; Morris R, in Journal of Neuroscience Methods 1984, 11 (1): 47-6*).* This behavioral model has been shown to be valid as a measure of hippocampaldependent spatial navigation and reference memory. For it, a two-day MWM protocol is reported, which is based on an initial series of training tests with the visible platform, followed by a memory test at 24 hours later, but with the hidden platform (Gulinello M., M. Gertner et al. in Behav. Brain Res., 196 (2009) 220-227). This protocol has been validated in elderly (15-18 months) triple transgenic (3xTG) mice. The criterion that is evaluated is the difference and/or the ratio of the escape latencies: that of the last test of the training with the visible platform and that of the first test with the hidden platform (24 hours later). Thus, animals that develop a correct spatial strategy move rapidly towards the hidden platform area, indicating that they keep their long-term memory capacity intact. In contrast, animals that take time to locate the hidden platform have long-term spatial memory failures, with higher escape latencies.

Figure 7 (Non-limiting Example 2) shows the results of the behavioral evaluation of compound **3** in the acute ESC model using the MWM. The results indicate that the mice administered with ESC have long-term spatial memory deficits, but not those treated with compound **3**. The arithmetic difference, as well as the ratio of the latency time of the last test of the training with the visible platform and that of the first test with the hidden platform shows that the group treated with compound **3** differs significantly from the group with ESC. In this way, the results of this experiment confirm those obtained in the previous experience on short-term spatial memory.

In these animals, the AChE activity ($\mu$mol/L/min/mg of protein) was determined *ex vivo,* in samples of soluble fraction of brain homogenates (Figure 8, Non-limiting Example 3). The results show that there was a significant increase

(p <0.001) in AChE activity in the ESC treated group compared to the control group. However, AChE activity was surprisingly significantly reduced in the group treated with **3** (p <0.05), compared to the ESC group, and as expected in the one treated with donezepil (p <0.01).

The chronic ESC model has recently emerged as another alternative in the experimental evaluation of new agents for the treatment of AD (Klinkenberg I and Blokland A, in Neurosci Biobehav Rev 2010, 34 (8): 1307-1350*).* In rodents, cognitive dysfunction is induced by administering serial doses of ESC (i.p.) for 10 consecutive days, at the rate of once daily administration. With this model, some characteristics that are manifested in the acute are maintained, and others appear that are better related to AD. Thus, a degeneration of the cholinergic system occurs such as; an increase in the activity/expression of acetylcholinesterase, a decrease in the activity / expression of the synthesis enzyme cholinoacetyltransferase and a deficit in the active consumption of choline at the presynaptic terminal level (Haider, S. et al. in Brain research bulletin, 2016, 127, 234-247). There is also an increase in the expression of the amyloid precursor protein (AβPP) and the presence of aggregates βA and hyperphosphorylated tau (Bihaqi SW, et al. in Indian J. Pharmacol. 2012, 44 (5): 593-598*;* Ramandeep K, et al. in Int J Prev Med Res. 2015. Vol. 1, No. 2, pp. 45-64).

Figure 9 (Non-limiting Example 4) shows the results of the long-term memory behavioral assessment of compound 3 in the chronic ESC model (1.5 mg/kg daily dose, 2 weeks). Compound **3** was given three days before ESC administration and during the next two weeks of ESC. The MWM results indicate the memory deficit of the mice administered with ESC. On the other hand, in the case of the groups administered with compound **3** (1 and 10 mg/kg), the ratio of the first escape latency of the test with the hidden platform (T7) and the last test with the visible platform (T6 ), significantly decreased compared to the ESC group. The results indicate that the 1 mg/kg dose behaves as the maximum effect dose. In summary, the results show that in this model a degeneration of the cholinergic system is induced, evidenced by the detriment of long-term spatial memory in MWM, which is prevented with the use of compound **3**.

It is recognized that glutamate receptor-mediated excitotoxicity may underlie the pathophysiology of a number of neurological abnormalities, including AD and Parkinson's disease (PD). Glutamate plays a central role in neurodevelopment, transmission and synaptic plasticity under normal physiological conditions. Also, this neurotransmitter is involved in memory and learning processes. In acute cytotoxic events such as hypoxia-ischemia or in chronic neurodegenerative diseases such as Alzheimer's and Parkinson's, glutamate concentrations rise in brain tissue, in response to different noxas, and this phenomenon triggers neuronal death, especially due to increased intracellular $Ca^{2+}$ concentrations. The compounds of Formula I have surprisingly been found, in *in vitro* tests, to have a neuroprotective effect against glutamate-induced excitotoxic damage, as shown in a non-limiting manner in Example 5 and Figure 10. Here, SH cells -SY5Y were pre-treated with compound **3** (1.8-60 μmol/L) for 24 hours, and subsequently co-incubated with the same concentrations of **3** and 60 mmol/L of glutamate (24 hours). Compound **3** significantly decreased (p <0.05) neuronal death at concentrations of 7.5 and 15 μmol/L. These results suggest that neuroprotection could differentially regulate mitochondrial metabolism.

Excitotoxic cell death is also affected by other receptors in the glutamatergic system. Thus, it is possible to experimentally induce the damage by the administration of kainic acid (KA) or α-amino-3-hydroxy-5-methyl-4-isoxazolepropionic acid (AMPA), both potent glutamate agonists (Wang Q, et al. in Mol Neurobiol. 2005, 31: 3-16). Rodent administration of KA is known to induce recurrent seizures, behavioral changes, and subsequent degeneration of neurons, specifically of the CA1 and CA3 regions of the hippocampus (Wang Q, et al. In Mol Neurobiol. 2005, 31: 3-16). The neuronal death induced by KA is accompanied by an increase in the activation of microglia and astrocytes, increasing the generation of inflammatory cytokines (Chen, Z. et al. In J. Neurobiol., 2005, 62 (2), 207-218). In addition, the activation of KA receptors produces the depolarization of the mitochondrial membrane and as a result, the alteration of intracellular $Ca^{2+}$ concentrations (Brorson, JR et al. in J. Neurosci., 1994, 14 (1), 187-197). Therefore, damage is induced in mitochondrial function (Wang, Q. et al. in Mol. Neurobiol., 2005, 31 (1-3), 3-16) and an increase in the generation of ROSs and ENOS, generating OE and nitrosative stress (NE), which induce cell death by their deleterious neuronal actions (Ueda, Y. et al. in Exp. Brain Res. 2002, 147(2), 219-226).

In our experience, in the animals of the KA control group, immobility, mouth and facial movements and rigid postures were observed within 10 to 20 minutes after KA injection, and in some cases tonic-clonic seizures of moderate intensity. During the next 20-60 minutes, nodding, repetitive movements and lifting and falling were observed, and subsequently, continuous lifting and falling until finally more severe tonic-clonic seizures were produced. However, in those treated with compound 3 (doses: 5 and 50 mg/kg) these effects were surprisingly manifested with less frequency and intensity (Figure 11, Non-limiting Example 6). Figure 11 shows the significant decrease in the total score (0-120 minutes) of the groups treated with **3** (5 and 50 mg/kg), the effect being greater with the 50 mg/kg dose.

The compounds of Formula **I** have not been formulated so far, so the pharmaceutical compositions of this invention are unique, which take into consideration the physico-chemical properties of these compounds and, in a singular way, the weight ratios of the ingredients in the compositions. Thus, the formulations presented guarantee the properties of these multi-target therapeutic agents with anticholinergic, antioxidant, antiglutamatergic and anti-inflammatory action. These formulations allow a sustained dose over time of effective plasma concentrations of the active ingredient, which ensure its efficacy, not described so far, and allow increasing adherence and tolerance through its oral, sublingual,

parenteral, transdermal and nasal administration.

The pharmaceutical formulations of the compounds of Formula **I**, their salts, hydrates, enantiomers, isomers, metabolites, prodrugs for administration to humans, of the present invention, include suitable excipients and can be in solid, semisolid, gelatinous or liquid form, such as tablets, capsules, suspensions, emulsions, parenteral solutions, transdermal patches, nasal solutions, mucoadhesives, among others. Typically, the formulation will contain 0.1 to 99%, preferably 1 to 80% active compound, together with the vehicle or components of the formulation. Excipients are liquid, solid or semisolid auxiliary substances, compounds of an organic and inorganic nature, of natural or synthetic origin, physiologically acceptable and commonly used in the formulation of active principles.

A pharmaceutical form of choice in this invention is tablets, which will be administered at least once a day and contain a therapeutically active ingredient selected from the group of compounds of Formula **I**, or a pharmaceutically acceptable salt thereof, and a pharmaceutically acceptable carrier of solid, semi-solid, gelatinous or liquid polymeric matrix. The dosage forms of the invention sustain the release of the therapeutically active agent between 2 to 24 hours when said form is exposed to aqueous solutions. After the administration of said formulation, the dissolution rate of the active principle is more than 80% between 6 and 18 hours, and the effective plasma level of the drug is maintained for 24 hours. The active ingredient is present in amounts of approximately 1 to 100 mg per formulation.

In the present invention, the tablets in particular use as polymers: polyethylene oxide, hydroxypropylmethylcellulose (HPMC), hydroxypropylcellulose (HPC), sodium carboxymethyl cellulose (CCNa), cellulose derivatives, Eudragit RS PO, polyvinylpyrrolidone or combinations thereof. In 24 hour modified release formulations, the polymer is present in amounts ranging from 5% w/w to 80% w/w, preferably from 10% to 70% w/w. In 12 hours modified release formulations, the polymer is preferably present at 5% w/w to 50% w/w. The formulations are prepared in the conventional manner described in the literature.

Figures 12, 13 and 14 show the controlled release profiles of three tablet formulations, which should not be considered as limiting of this invention (Examples 7, 8 and 9), for obtaining tablets of the active principles : (N-[4-(1-naphthylamino)-4-oxobutanoyl]-β-alanine **(1),** 6-{[4-(1-naphthylamino) - 4-oxobutanoyl]amino}hexanoic acid) **(2)** and methyl(2-{[4-(1-naphthylamino)-4-oxobutanoyl]amino}ethyl)dithiocarbamate **(3).** These formulations contain 10 mg of active principle, and other additives such as starch as a binder, microcrystalline cellulose as a disintegrant, lactose as a diluent, silicon dioxide as a gliding agent and magnesium stearate as a lubricant, in different proportions and it was used as polymeric matrices: HMPC, HPC and Eudragit RS PO. Figures 12, 13 and 14 show that in the three formulations there are two release phases dependent on the pH of the medium. Thus, after 60 minutes of exposure of the tablets (HMPC, HPC and Eudragit RS PO), to a non-enzymatic acid medium, the release of the active principles from the matrix was not significant, with less than 1% of the principle released. On the other hand, when the tablets of the three formulations were exposed to a buffer solution of pH 7.5, the release process of the active principles increases gradually, obtaining an almost complete delivery, more than 96%, to the medium during 24 hours. This release occurs for the three active principles exemplified as non-limiting.

These release profiles of the three formulations, presented with a non-limiting nature of the invention, clearly show that when the pH changes there is an increase in the slope in the dissolution curve, which means that there is an overtime sustained release of the active principle. The implication of this release pattern for human use, suggests that the release occurs in two stages where there is a gradual and moderate increase in the concentration of the active principle in the blood, thus reducing unwanted side effects. This is particularly effective in avoiding possible gastric side effects as only about 1% of the active ingredient content is released in the stomach. Beyond this, a reliable slow release, generally at long dosing intervals, improves the pharmacological effect in the patient thereby increasing adherence to treatment and tolerance.

In summary, the tablet formulations of the Formula **I** compounds of this invention have an optimal release profile. It uses accessible polymeric matrices so that the tablets can be produced in a simple and economical way with the incorporation of physiologically compatible components, which provides extended release compositions in two phases, which facilitate the dosage, without producing adverse side effects.

The transdermal system developed in this invention is made up of the following successive layers: a) lipophilic protective outer layer; b) layer containing the active principle of Formula **I**, in the form of a gel, suspension, emulsion, or other; where the compound is incorporated into a matrix (which can be acrylic), which ensures its effective storage; c) bifacial controlling membrane that releases the active principle; d) release module, which includes an active principle reservoir and a drug release controller system and is generally made up of a polymeric material (carboxymethylcellulose (CMC), ethylcellulose, gelatin, methylcellulose, starch, etc; synthetic elastomers: polybutadiene, polyisoprene, neoprene, polysiloxane, etc; synthetic polymers: polyvinyl alcohol (PVA), polyethylene, polystyrene, polyurethane, polyvinyl pyrrolidone (PVP), etc. e) hypoallergenic adhesive layer, which allows the patch to be fixed by pressure and contains polymers acrylics, hypoallergenic silicone, resins, mineral oils, polyisobutylene, etc. and f) internal protective film, which is removed before applying the system to the skin. The coating of these topical patches ensures a continuous and gentle release of the drug, through the skin into the bloodstream; without adverse reactions. Two types of patches are declared, according to their size and dosage capacity. One of an area of 5 $cm^2$, dose of 5 mg of active principle and release of 4

mg/day of compound. Another with doubled proportions and capacity. Therapy begins with a daily dose of the smaller patch, and after good tolerance and a minimum of 4 weeks, the larger patch (10 $cm^2$, 10 mg/24 h) is applied.

The nasal formulation of the compounds of Formula I, their salts, hydrates, enantiomers, isomers, metabolites, prodrugs for their administration to humans, of the present invention employs different components that function as solvent, bioadhesive, preservative, antioxidant and surfactant/humectant.

Non-limiting bioadhesive excipients can be: hydroxypropylmethylcellulose (HMPC), hydroxypropylcellulose (HPC), methylcellulose (MC), carboxymethylcellulose (CMC), all of them with concentrations from 0.5 to 7% and derivatives of polyacrylic acid (carbol) from 0.01 to 1.5%. PEG is used as a solvent for the active principles. Bioadhesive polymers have, in a non-limiting manner, a viscosity of 10 to 60 mPas, with the aim of increasing the residence time in the nasal cavity. As an antimicrobial preservative excipient, benzalkonium chloride from 0.005 to 0.5% or in combination with disodium EDTA, in concentrations from 0.002 to 0.2% and propyl paraben from 0.005 to 0.05%, is preferably used. The pH was adjusted between 6 and 7.5 using isotonic solutions of phosphate or citrate buffers. The isotonizing agents can be: sodium chloride, mannitol, sorbitol, glucose, among others. The preferred antioxidant used is tocopherol from 0.05 to 2%. Glycerol from 3 to 6%) and Tween 80 from 0.1 to 0.4% are used as wetting agents, which allow to increase permeability. The solvent used in all cases was water for injection.

Intranasal administration is considered a method of delivering therapeutic agents to the central nervous system (CNS). Drug release occurs within minutes along the olfactory and neural trigeminal pathways, extracellular pathways that do not require the drug to bind to any receptor or axonal transport system (Thorne, RG et al. in Neuroscience. 2002, 127, 481-496).

Figure 15 shows the results of the behavioral evaluation (Y-maze test) of the effect of the compound **3** nasal formulation administration on the amnesia induced by the acute administration of ESC. As can be seen, there are no differences between the control groups, which indicates that the formulation used does not affect the performance of the animals in the Y-maze test. As expected, a significant decrease ($p < 0.001$) of the scan time in the novel arm of ESC-treated animals was observed compared to both control groups. However, the animals in the group treated with compound **3** show similar values to those of the control groups (saline and vehicle), indicating that **3** prevents ESC-induced amnesia. Figure 16 shows the results in the Object Discrimination Test (ODT). No significant differences were observed in the discrimination index (DI) of the novel object between the control groups and the group treated with **3**. On the other hand, in the group administered with ESC, a drastic decrease of the DI was observed ($p < 0.001$).

The results of this experiment show that the acute administration of ESC induces cognitive dysfunction both in the Y-maze model and in ODT, analogously to that observed by other authors (de Bruin N, et al., J Neurodegener Dis. 2015; 2015: 242505. Doi: 10.1155 / 20151242505). Along with this, intranasal administration of compound **3** prevents cognitive dysfunction in both models, which is consistent with previous results for this compound, administered orally, in the acute and chronic ESC model. The intranasal administration of **3** makes it possible to prevent the amnesic effects of ESC at lower doses than those used in the oral route.

The following examples and figures, which should not be interpreted in any way as limiting the present invention, illustrate the multi-target target action on the different mechanisms of the cholinergic, glutamatergic and mitochondrial systems of the pharmaceutical formulation of the compounds of Formula I, their salts, hydrates, enantiomers, isomers, metabolites, and prodrugs for administration to humans.

Description of the content of the figures

**Figure 1:** Structures and physicochemical properties of some of the compounds of Formula I selected for the non-limiting examples of this invention.

**Figure 2:** Effect of the compound of Formula I (N-[4-(1-naphthylamino)-4-oxobutanoyl]-β-alanine (**1**) on the membrane potential of mitochondria isolated from rat liver.

**Figure 3:** Effect of the compounds of Formula I (N-[4-(1-naphthylamino)-4-oxobutanoyl]-β-alanine (**1**) on the swelling of mitochondria isolated from rat liver. *** $p < 0.001$ Comparison vs control by Student's t test, $p < 0.05$ was considered as a statistically significant difference.

**Figure 4:** Effect of compounds of Formula I (N-[4- (1-naphthylamino)-4-oxobutanoyl]-β-alanine (**1**) on the production of reactive oxygen species in mitochondria isolated from rat liver. *** $p < 0.001$ Comparison against EGTA group using Student's t test, $p < 0.05$ was considered as a statistically significant difference.

**Figure 5:** Anticholinergic modulating effect of the compound methyl (2-{[4-(1-naphthylamino)-4-oxobutanoyl]amino}ethyl)dithiocarbamate (3) in a model of memory loss induced by an acute administration of scopolamine (ESC). Short-term spatial memory in the Y-maze after the acquisition session of the control, ESC and treated groups at single doses with 3 (0.1, 1, 10 and 20 mg/kg, i.p.). * $p < 0.05$, ** $p < 0.01$, *** $p < 0.001$. One-way analysis of variance followed by Turkey's *post-hoc* test.

**Figure 6:** Anticholinergic modulating effect of the compound methyl (2-{[4-(1-naphthylamino)-4-oxobutanoyl]amino}ethyl)dithiocarbamate (3) in a model of memory loss induced by an acute administration of scopolamine (ESC). Time of permanence in the novel arm, after the training session of the control groups, ESC and treated with **3** at doses of 0.1, 1, 10 and 20 mg/kg. * $p < 0.05$, comparison vs control group ≈ $p < 0.05$, comparison vs ESC. One-way analysis of variance followed by Turkey's *post-hoc* test.

**Figure 7:** Anticholinergic modulating effect of the compound methyl (2-{[4-(1-naphthylamino)-4-oxobutanoyl]amino}ethyl)dithiocarbamate (3) in a model of memory loss induced by an acute administration of scopolamine (ESC). Upper panel: Performance in the MWM in the two-day protocol of the different experimental groups. Lower panels: Long-term spatial memory, escape latency of the last trial with the visible platform (D1T4); escape latency of the first trial with the visible platform (D2T1). ** $p < 0.01$, *** $p < 0.001$, comparison vs ESC group. ≈ $p < 0.05$, comparison vs control group. One-way analysis of variance followed by Turkey's *post-hoc* test.

**Figure 8:** Anticholinergic modulating effect of the compound methyl (2-{[4-(1-naphthylamino)-4-oxobutanoyl]amino}ethyl)dithiocarbamate (3) in a model of memory loss induced by an acute administration of scopolamine (ESC). Anticholinesterase activity in soluble fractions of animal brain homogenates from the acute ESC model of the control group, ESC-treated group, and methyl (2-{[4-(1-naphthylamino)-4-oxobutanoyl]amino}ethyl)dithiocarbamate (3) and donezepil. The bars correspond to the mean value ± SEM, n = 10 animals/group. *** $p < 0.001$, comparison vs control group. ≈ $p < 0.05$, ≈≈ $p < 0.01$, comparison vs ESC group. A $p < 0.05$ was considered a statistically significant difference. Statistical test used: one-way analysis of variance followed by Turkey's *post-hoc* test.

**Figure 9:** Anticholinergic modulating effect of the compound methyl (2-{[4-(1-naphthylamino)-4-oxobutanoyl]amino}ethyl)dithiocarbamate (3) in a model of memory loss induced with the use of chronic Scopolamine. *** $p < 0.001$, comparison vs control group. ≈ $p < 0.05$, ≈≈ $p < 0.01$, comparison vs ESC group. One-way analysis of variance followed by Turkey's *post-hoc* test.

**Figure 10:** Neuroprotective effect of the compound methyl (2-{[4-(1-naphthylamino)-4-oxobutanoyl]amino}ethyl)dithiocarbamate (3) on the excitotoxic damage induced by glutamate, in the cell line SH-SY5Y. SH-SY5Y cells were treated with different concentrations of compound **3** (1.8-60 μmol/L), for 24 hours, then the medium was replaced by new medium containing the compound again and glutamate 60 mmol/L. Controls: untreated cells (NT) and glutamate (Glut). Viability was determined by the MTT test. Methyl (2-{[4-(1-naphthylamino)-4-oxobutanoyl]amino}ethyl)dithiocarbamate significantly decreased cell death generated by glutamate at concentrations (7.5 and 15 μmol/L). Each bar represents the mean ± SEM, n = 3. A $p < 0.05$ was considered a statistically significant difference.

**Figure 11:** Neuroprotective effect of the compound methyl (2-{[4-(1-naphthylamino)-4-oxobutanoyl]amino}ethyl)dithiocarbamate (3) on seizures induced by icv administration of kainic acid, expressed as final scores (0-120 minutes) of the different groups of animals. The bars represent the mean value ± SEM, n = 7 per group. * $p < 0.05$; ** $p <= .001$. Comparison vs control group. One-way analysis of variance followed by Turkey's *post-hoc* test. A value of $p < 0.05$ was considered a statistically significant difference.

**Figure 12:** Release profiles of Formulation 1 of tablets of the compounds: (N-[4-(1-naphthylamino)-4-oxobutanoyl]-β-alanine (1) and methyl (2-{[4-(1-naphthylamino)-4-oxobutanoyl]amino}ethyl)dithiocarbamate (3), without enzymes at pH 1.2, at pH 7.5 and changing from pH 1.2 to 7.5, for 18 hours.

**Figure 13:** Release profiles of Formulation 2 of tablets of the compounds: 6-{[4-(1-naphthylamino)-4-oxobutanoyl]amino}hexanoic acid) (2) and methyl(2-{[4-(1-naphthylamino)-4-oxobutanoyl]amino}ethyl)dithiocarbamate (3), without enzymes at pH 1.2, at pH 7.5 and changing from pH 1.2 to 7.5, for 18 hours.

**Figure 14:** Release profiles of Formulation 3 of tablets of the compound: methyl (2- {[4-(1-naphthylamino)-4-oxobutanoyl]amino}ethyl)dithiocarbamate (3), without enzymes at pH 1.2, at pH 7.5 and change from pH 1.2 to 7.5, for 18 hours.

**Figure 15:** Effect of the administration of single intranasal doses (7.2 mg/kg) of compound **3** on the amnesia induced

by the acute administration of scopolamine (1.5 mg/kg, ip) in the Y-maze test. The bars represent the mean value ± SEM of the time spent in the novel arm, n = 10 animals/group. *** p <0.001; comparison vs saline control group and vehicle control. ≈≈ p <0.01. Comparison vs ESC group. Simple ANOVA test followed by Turkey's multiple comparisons test.

**Figure 16:** Effect of the administration of single doses by intranasal route (74 μg/kg) of compound **3** on the amnesia induced by the acute administration of scopolamine (1.5 mg/kg., i.p.) in the object discrimination test. The bars represent the mean value ± SEM of the discrimination index, n = 10 animals/group. *** p <0.001; comparison vs saline control group and vehicle control. ≈≈≈ p <0.001. Comparison vs scopolamine group. Simple ANOVA test followed by Turkey's multiple comparisons test.

Examples are set out below for three of the compounds of Formula I, which should not be construed as limiting the present invention in any way.

**Example 1. Evaluation of the effect of the Formula 1 compound (N-[4-(1-naphthylamino)-4-oxobutanoyl]-β-alanine (1) on the membrane potential, swelling and production of reactive oxygen species in mitochondria isolated from rat liver.**

Isolation of rat mitochondria: Mitochondria were isolated by the classical method of differential centrifugation from the liver of male Wistar rats (Mirandola SR, et al., in J Neurosci Res 2010; 88: 630-39).

Incubation procedures: Mitochondria were energized with 5 mmol/L potassium succinate (plus 2.5 μmol/L rotenone) in standard incubation medium containing 125 mmol/L sucrose, 65 mmol/L KCl and 10 mmol/L of HEPES-KOH, pH 7.4, at 30 °C.

Mitochondrial assays

Mitochondrial membrane potential and ROS were determined by spectrophotometry, using as fluorescent probes, respectively, safranin (10 pmol/L) (Zanotti A., and Azzone GF in Archives of Biochemistry and Biophysics 1980, 201 (1), 255-265j and Amplex Red (Molecular Probes, OR, Eugene) in the presence of horseradish peroxidase (1 IU/mL) (Votyakova and Reynolds, in J Neurochem. 2001, 79 (2), 266-77). Measurements were made in a Hitachi fluorescence spectrophotometer, model F-4500 (Tokyo, Japan) at Xexc 495 / Xem 586 nm (safranin) and at Xexc 563/aem 587 nm (Amplex Red). These experiments were carried out in the presence of 0.1 mmol/L of EGTA and the evaluated compound was N-[4-(1-naphthylamino)-4-oxobutanoyl]-β-alanine (**1**) in a concentration range of 0.1 to 100 μmol/L. Mitochondrial swelling was estimated spectrophotometrically from the decrease in absorbance at 540 nm, using a Hitachi spectrophotometer, Model U-2910 (Japan).

Data analysis: In all cases, the normal distribution and homogeneity of variances of the data were evaluated using the Kolmogorov Smirnov and Levene tests, respectively. The comparison of the mitochondrial functionality variables was carried out by means of a Student's t-test. We worked with a significance level of 0.05 and the Statistica 8.0 program (StatSoft Ink) was used for data analysis. The graphical representations of the results were made in the GraphPad Prism 5.0 program (GraphPad Software, California, USA).

**Example 2. Evaluation of the anticholinergic modulating effect of the compound methyl (2- [4-(1-naphthylamino)-4-oxobutanoyl]amino}ethyl)dithiocarbamate (3) in a model of memory loss induced with the acute use of Scopolamine.**

Animals

Young male OF-1 mice (20-25 g), supplied by the National Center for the Laboratory Animals Production (CENPALAB, Cuba), were used. Before the experiment, the animals were adapted for seven days to laboratory conditions (controlled temperature 25 ± 2°C, relative humidity 60 ± 10% and light/dark cycles of 12 h). During this period, and during the experiment, the animals received *ad libintum* water and a standard diet for rodents supplied by CENPALAB. All procedures were carried out in accordance with the European Commission guidelines for the use and care of laboratory animals and approved by the Committee for the Care of Research Animals. The minimum number of animals and duration of observations required to obtain consistent data was used.

*Experimental design.*

At the end of the adaptation period, the animals were randomly distributed according to their body weights in

5 experimental groups of 12 animals per group. Scopolamine hydrobromohydrate (ESC, Sigma Aldrich) was used to induce cognitive deficit, which was dissolved in physiological saline and administered intraperitoneally (ip) at a dose of 1.5 mg/kg, 30 minutes before the test ( Miranda HF et al. in. J. Biomed. Sci., 2014 21 (1): 62-62). The doses of compound **3** used were 0.1, 1, 10 and 20 mg/kg of body weight. The compound was formulated using carboxymethyl cellulose (CMC). The administration scheme was designed as follows:

Group 1 (negative control) with normal cognitive function. Two administrations with an interval of 30 minutes with vehicle (5% CMC) by oral route and saline solution by i.p. route, respectively.

Group 2 (positive control) with cognitive dysfunction. Two administrations with an interval of 30 minutes, the first with vehicle (5% CMC), the second with ESC 1 mg/kg.

Group 3, 4, 5 and 6 treated with **3** (0.1, 1, 10 and 20 mg/kg). Two administrations with an interval of 30 minutes, the first with **3** at the dose corresponding to each group, the second with ESC 1 mg/kg. The behavioral evaluation was carried out 30 minutes after this second administration.

Behavioral tests

Y Maze

The study of short-term spatial memory was carried out through the study of novel spatial preference, using the Y-maze behavioral paradigm (40 cm long x 9 cm wide x 16 cm high) with extra-labyrinthine tracks around of the maze. The test consisted of two trials separated by an interval of 2 hours. In the first training test (acquisition), the mice were placed in the center of the maze and facing the end of one of the arms whose selection was random. After being positioned, they were allowed to explore the maze for 10 minutes with one of the arms closed (novel arm). At the end of this test, the mice were returned to their original cage until the second test (recovery), during which they could freely explore the three arms of the maze for 5 minutes. The total residence time (exploration) in each arm was measured and analyzed from video recordings. The entrance to the arm was defined as the entrance of the four legs of the animal in said arm. The residence time (sec) in each arm during the recovery test of the learned task was used for the statistical analysis. The maze was cleaned with 70% ethanol between runs. All mice were transported to the behavioral testing room in their home cages at least 1 day prior to testing. The experiments were carried out between 8:00 am and 12:30 pm.

Morris Water Maze (MWM).

The experience was carried out for two days; a first day of training and a second day of testing. All mice were transported to the behavioral testing room in their home cages at least 1 day prior to testing and experiments were conducted between 8:00 am and 12:30 pm. The animals were trained according to the criteria in a series of visible platform tests on day 1 (D 1) in a pool of 120 cm in diameter and 51 cm deep with the water temperature of 24 ± 2 °C, where high-contrast visuals signals were placed on the wall in each quadrant and external signs in the room. The training phase comprised four tests with the visible platform (V1-V4) and the last test was considered as its basal latency of escape after habituation. This was designated D1V4 (day 1, test visible platform 4). The training of the animals was staggered in time so that each mouse had the same interval between tests, that is, 30 ± 10 minutes between each test and was started each time from different positions of the labyrinth selected randomly. In order to make the platform visible, it was identified with a high contrast object with respect to the bottom of the tank. Animals that could not escape within 1 minute were manually guided onto the platform and allowed to remain on it for 5-10 seconds before being removed. Subsequently, they were dried and placed in their respective cages lit with an incandescent light to avoid hypothermia. 24 hours after the last visible platform test (D2), the animals were evaluated in a series of three tests with the hidden platform (T1-T3). The tests were carried out every 30 minutes and each test had a maximum of 1 minute in duration. For all tests the platform remained in the same place.

Statistical analysis.

All data are expressed as the mean ± standard error of the mean (SEM). The Morris water maze data was analyzed in two ways. Escape latencies in the visible platform training phase were analyzed using a two-factor ANOVA (treatment x trial). For the study of long-term memory, the last escape latency value of the training phase with the visible platform (D1V4) and the first value of the test with the hidden platform performed 24 h later (D2T1) were used. Mice with intact long-term memory capacity should quickly reach the hidden platform in the first test. A scoring system was generated with the difference D2T1-D1V4 and with the ratio D2T1 / D1V4. The D2T1-D1V4 score for a successful subject

should be close to 0, and the D2T1 / D1V4 score around 1 or less. Higher values indicate a longer escape latency after 24 h, which implies a deficit in the spatial performance of the animals. This comparison was made using a one-way analysis of variance, followed by Turkey's *post-hoc* test. Analysis of the Y-maze data was also performed using the same test above. A $p < 0.05$ was considered a statistically significant difference. Statistical analysis was performed using GraphPadPrism version 5 (GraphPad Software, Inc., San Diego, USA).

**Example 3. Evaluation of the anticholinergic modulating effect of the compound methyl (2-{[4-(1-naphthylamino)-4-oxobutanoyl]amino}ethyl)dithiocarbamate (3) in a model of memory loss induced with the acute use of Scopolamine through AChE activity in whole brain homogenates.**

AChE activity in the brain of mice was determined according to the colorimetric assay of Ellman et al. (Ellman GL, et al. In Biochem Pharmacol., 1961; 7: 88-95) adapted to determine enzyme activity in brain homogenate supernatant. Briefly the procedure was as follows; at the end of the behavioral experiments, the mice were anesthetized and decapitated to quickly remove the brains, which were placed on an ice-cold plate. They were weighed and homogenized (1/10, w/v) in cold 10 mmol/L Tris-HCl buffer, pH 7.2, containing 160 mmol/L sucrose. The homogenates were centrifuged at 10,000 XG for 10 minutes at 4 ° C, and the resulting clear supernatants were used as enzyme sources, which were aliquoted (n = 10 per group) and stored at -20 ° C. The assay was performed in 96-well microtiter plates using Ellman's reagent (DTNB 10 mmol/L), 50 μL homogenate and 10 μL Acetylcholine iodide (AChI, 20 mmol/L) (final volume 200 pL). The absorbance of the yellow hydrolyzate of the 5-thio-2-nitrobenzoic acid dianion (molar extinction coefficient: 13 700 $mol/L^{-1}$ $cm^{-1}$), was measured at 412 nm, for 20 minutes with an interval of 1 minute. AChE activity was performed in triplicate and was expressed in nmol of hydrolyzed AchI per minute per mg of protein. Protein concentrations were determined by the modified Lowry method (Markwell MAK et al. In Anal. Biochem. 1978, 87 (1): 206-210).

Statistical analysis.

All data are expressed as the mean ± standard error of the mean (SEM). The comparison of the cholinesterase activity values was carried out by means of a one-way analysis of variance, followed by the Turkey *post-hoc* test. Statistical analysis was performed using GraphPadPrism version 5 (GraphPad Software, Inc., San Diego, USA).

**Example 4. Evaluation of the anticholinergic modulating effect of the compound methyl (2-{[4-(1-naphthylamino)-4-oxobutanoyl]amino}ethyl)dithiocarbamate (3) in a model of memory loss induced with the chronic use of Scopolamine.**

Animals:

Male C57BL / 6 mice (20-25 g), supplied by the National Center for the Laboratory Animals Production (CENPALAB, Cuba) were used. During the experiment, the animals received *ad libitum* water and standard diet supplied by CENPALAB. All procedures were carried out in accordance with the European Commission guidelines for the use and care of laboratory animals and approved by the Committee for the Research Animals Care. The minimum number of animals and duration of observations required to obtain consistent data was used.

Experimental design.

At the end of the adaptation period, the animals were randomly distributed according to their body weights in five experimental groups of 12 animals each. For the induction of cognitive deficit, ESC (Sigma Aldrich) was used, which was dissolved in physiological saline and administered daily intraperitoneally (ip) at a dose of 1.5 mg/kg for 2 weeks, in the morning (Park JH, et al. In J Mol Neurosci. 2016; 59 (4): 579-589). Compound **3** was prepared as a suspension, in 0.5% soluble starch (Merck Millipore), and administered orally by intragastric cannula at a daily dose of 0.1, 1, 10 mg/kg of body weight (0.01 mL/kg). The experimental groups were:

Group 1 (negative control) with normal cognitive function. Daily administrations of the vehicle: starch 0.5% (oral) and physiological saline solution (i.p.) for two weeks.

Group 2 (positive control) with cognitive dysfunction. Daily administrations of ESC 1.5 mg/kg for two weeks and vehicle (starch 0.5% orally).

Group 3, 4, 5 the administration of compound **3** was started four days before the start of the administration of ESC. The animals in each group were treated with 3 orally at doses of 0.1, 1 and 10 mg/kg, respectively. Daily administration

of ESC (1.5 mg/kg) was carried out for two weeks. At the conclusion of this treatment, the behavioral assessment was started.

Morris Water Maze (MWM): Analogous to Example 2, the two-day MWM test was used to determine long-term spatial memory. In this case, as a 1.35 m diameter tank was used, six tests were carried out during the training period (D1V1 to D1V6), and three tests at 24 h later (D2T1 to D2T3). In this way, long-term spatial memory was determined by the quotient of the escape latency values D2T1 / D1V6.

Statistical analysis.

For the analysis of the long-term memory data, a statistical procedure similar to that of non-limiting Example 2 was carried out, with the help of the GraphPadPrism version 5 program (GraphPad Software, Inc., San Diego, USA).

**Example 5. Neuroprotective effect of the compound methyl (2 - {[4-(1-naphthylamino) -4-oxobutanoyl] amino} ethyl) dithio carbamate (3) on the excitotoxic damage induced by glutamate, in the cell line SH-SY5Y.**

Compound 3 was dissolved in DMSO at a concentration of 100 mmol/L, and subsequently aliquoted and stored at -20 °C. RPMI 1640 (1X) + GlutaMax medium (Gibco Laboratories Life Technologies, Inc .; Rockville, MD, USA), containing HEPES buffer (25 mmol/L) and L-glutamine (2 mmol/L), was supplemented with 10 % Fetal Bovine Serum (FBS), 1% sodium pyruvate (100 mmol/L), 1% antibiotic mixture penicillin (10,000 U/mL) -treptomycin (10 mg/mL), 1% gentamicin ( 10 mg/mL), Trypsin-EDTA (0.25%) and phosphate buffered saline (1X DPBS). 3- (4,5-dimethyl-2-thiazoyl) -2,5-diphenyltetrazole (MTT) bromide, dimethylsulfoxide (DMSO) and glutamic acid were Sigma Chemical Co. (St. Louis, MO, USA).

Cell culture: SH-SY5Y human neuroblastoma cells (ATCC CRL-2266) were grown in T-75 $cm^2$ flasks with RPMI 1640 culture medium containing HEPES buffer (25 mmol/L) and L-glutamine (2 mmol/L) supplemented with 10% fetal bovine serum (FBS), previously inactivated by heat, 1 mmol/L sodium pyruvate, benzylpenicillin antibiotic mixture (100 U/mL) and streptomycin (100 μg/mL). In addition, gentamicin (100 μg/mL) was added, then called complete medium (CM). The culture was incubated in a humid atmosphere of 5% $CO_2$, 95% air and a temperature of 37°C. The medium was changed every two days and the cells were subcultured every five days by trypsinization at a density of 4-5 $\times 10^6$ cells/75 $cm^2$ flask for experimental work and / or subculture of a new generation of cells for later use.

Methyl (2-{[4-(1-naphthylamino)-4-oxobutanoyl]amino}ethyl)dithiocarbamate (**3**) cytotoxicity: SH-SY5Y human neuroblastoma cells were cultured in RPMI 1640 (CM) incubated at 37 °C in an atmosphere of $CO_2$ 5% and air 95%. When they reached a confluence between 80-90%, they are trypsinized with a 0.25% Trypsin EDTA mixture (1 mL/25 $cm^2$) for 2-3 minutes to detach the cells. Then this suspension is neutralized with the same volume of MC. Subsequently, they were centrifuged at 112 X G for 8 minutes, the supernatant was removed by decantation and the precipitate was resuspended in 1 mL of complete medium for counting in the hemocytometer.

Once the cell density was known, they were seeded in 96-well culture plates (200 μL in each well) at a concentration of 80 $\times 10^4$ cells/well, after 24 hours of incubation, 100 μL of supernatant was removed in each well and subsequently added. 100 μL of compound **3,** prepared in CM at different concentrations (0.93, 1.87, 3.75, 7.5, 15, 30, 60 and 100 μmol/L) and incubated again for 24 hours under conditions of 5% $CO_2$, 95% air at 37°C. After the incubation time had elapsed, cell viability was determined by the MTT assay.

Neuroprotection of methyl (2 - {[4- (1-naphthylamino) -4-oxobutanoyll amino} ethyl) dithium carbamate against glutamate-induced neuronal death: To evaluate the neuroprotective effect of compound **3,** the cell line SH-SY5Y was cultured until reaching a confluence between 80-90%, the cells were tripzinized and subsequently counted in the hemocytometer. Once the cell density was known, they were seeded in a 96-well culture plate (200 μL in each well) at a concentration of 80 $\times 10^4$ cells/well. Subsequently, after 24 hours of incubation at 37°C in a humid environment with 5% $CO_2$, 100 μL of supernatant is eliminated in each well and 100 μL of compound **3** prepared in complete medium is added at different concentrations (0.93; 1.87; 3.75; 7.5; 15; 30; 60 μmol/L) and incubated again for 24 hours under conditions of 5% $CO_2$, 95% air at 37°C. Once the incubation time has elapsed, 100 μL of supernatant is eliminated in each well and 100 μL of the freshly prepared compound **3** mixture is added at the same concentrations as the previous day and 60 mmol/L of freshly prepared glutamic acid in complete medium and incubated again for 24 hours under conditions of $CO_2$ 5%, air 95% at 37 ° C. After the incubation time, cell viability was determined by the MTT assay.

Cell viability. MTT assays: Cell viability was measured through formazan blue products obtained from the enzymatic transformation of MTT (3-(4,5-dimethyl-2-thiazolyl)-2,5-diphenyl-2Htetrazoliumbromide) by mitochondrial dehydrogenases, which are active only in viable cells. The assay was carried out in 96-well plates, the cells were exposed to glutamate as a stressor, to compound **3** or to both at different times and concentrations. After being subjected to the different treatments, the MTT was added (freshly prepared at 5 mg/mL in MC), 50 pL/well was added and incubated for 4 hours in an atmosphere of 5% $CO_2$, 95% air at $37^0$C. Then 100 μL of isobutylalcohol (SDS 10%, 2-butanol 50%, 0.25

μL HCl 2 mmol/L) was added to each well to solubilize the formazan crystals, and the optical density of each well was measured before 30 minutes at 570 nm in a Clariostar spectrophotometer (BMG Labtech). The correction of the OD values was carried out at 690 nm. Three replications were carried out for each experiment. The results were expressed as the percentage of optical density of each experimental variant in relation to the Optical density obtained for the group of cells not treated with glutamate.

**Example 6 Neuroprotective effect of the compound methyl (2-{[4-(1-naphthylamino)-4-oxobutanoyl]amino} ethyl)dithiocarbamate (3) on seizures induced by the icv administration of kainic acid.**

Experimental animals and groups.

Young male OF-1 mice (20-25 g), supplied by the National Center for the Laboratory Animals Production (CENPALAB, Cuba) were used. The conditions of adaptation and maintenance of the animals before carrying out the experiment were similar to those developed in non-limiting Example 2. The mice were divided into 5 experimental groups (7 animals group) distributed in: a control group (PBS 2 pL, icv), a seizure control group (KA 2 pL, icv) and three groups treated orally by gastric cannulation with compound **3** at doses of 1, 5, 50 mg/kg (0.01 mL/kg) and KA 2 pL, icv. Compound **3** was prepared as a suspension in 5% carboxymethylcellulose (CMC) vehicle. The administration **of 3** and the vehicle in the control groups was carried out 1 h before the icv injection of KA. All animals were administered with KA (Sigma Aldrich) prepared in PBS at a concentration of 0.5 mg/mL.

KA icv administration.

For the icv administration of KA the mice were anesthetized with 685.71 mg/kg with chloral hydrate i.p. and placed on a dual manipulator. The stereotaxic frame and the skull were exposed. Stereotaxic coordinates for the lateral ventricle were precisely measured as: anteroposterior -0.8 mmol/L, lateral ± 1.0 mmol/L and dorsoventral -3.0 mmol/L relative to bregma and ventral dura with the tooth bar at 0 mmol/L. A burr hole was made in the skull by automatic microperforation attached to an arm of the stereotaxic apparatus. Through the hole, a 10 μL 28-gauge Hamilton® syringe was placed, attached to another arm of the stereotaxic apparatus, and the piston of the syringe was manually lowered into the right lateral ventricle, emptying its contents for 3 minutes.

Behavioral observations

Seizure scoring was performed according to the Racine scale (R.J. Racine, in Clin. Neurophysiol. 1972, 32 281-294) using the following measurement criteria: (1) immobility, mouth and face movements; (2) forelimb extension and / or tail extension, stiff posture; (3) repetitive movements, head rocking; (4) steepness and fall; (5) continuous steepness and falls; (6) severe tonic-clonic seizures; (7) death. Each animal was observed by two independent observers and the behaviors were quantified for 2 hours. The sum of the accumulated score for each animal during this time interval was used for statistical analysis.

Statistical analysis

All data are expressed as the mean ± standard error of the mean (SEM), and were analyzed using a one-way analysis of variance followed by Turkey's *post-hoc* test. For comparison between groups, the mean of the accumulated score for each experimental group was used 2 h after the icv administration of KA was performed. A $p < 0.05$ was considered a statistically significant difference. Statistical analysis was performed using GraphPadPrism version 5 (GraphPad Software, Inc., San Diego, USA).

**Example 7. Formula 1 for the manufacture of tablets with hydroxypropylmethylcellulose (HPMC) of the active principles N-[4-(1-naphthylamino)-4-oxobutanoyl]-β-alanine (1) and methyl(2-{[4-(1-naphthylamino)-4-oxobutanoyl]amino} ethyl)dithiocarbamate (3)**.

All components were weighed according to the quantities required in the formula. The active ingredient and the polymeric matrix were sieved through a No. 40 sieve mesh and subsequently mixed for five minutes at 30 rpm. Then, every 15 minutes, the rest of the components were added at a speed of 50 rpm, until a homogeneous mixture was obtained. The mixture is then compressed on a high-speed rotary machine, using flat, beveled and unslotted dies.

The tablets obtained were smooth and uniform, the weight variation is less than 1%, of adequate hardness (4.51 ± 0.37 kgf), with good abrasion resistance, adequate disintegration time and the content of the active principle is found between 90 and 110%.

The statistical treatment of the release profiles of the active principle from the hydrophilic matrices under study was carried out using the Origin 5.0 software with a confidence level of 95%.

Manufacturing formula 1 per tablet:

| Components | Weight (mg) | % |
|---|---|---|
| Active principle | 10 | 10 |
| HPMC | 25 | 25 |
| Lactose monohydrate | 9 | 9 |
| Starch | 21 | 21 |
| Microcrystalline cellulose | 32 | 32 |
| Silicon dioxide | 1.5 | 1.5 |
| Magnesium stearate | 1.5 | 1.5 |
| Tablet Weight (mg) | 100 | 100 |

**Example 8. Formula 2 for the manufacture of tablets with hydroxypropylcellulose (HPC) of the active principles N-[4-(1-naphthylamino)-4-oxobutanoyl]-β-alanine (1) and methyl(2-{[4-(1-naphthylamino)-4-oxobutanoyl]amino}ethyl)dithiocarbamate (3).**

All components were weighed according to the quantities required in the formula. The active principle and the polymeric matrix were sieved through a No. 40 sieve mesh and subsequently mixed for 5 minutes at 30 rpm. Then, every 15 minutes, the rest of the components were added at a speed of 50 rpm, until a homogeneous mixture was obtained. The mixture is then compressed on a high-speed rotary machine, using flat, beveled and unslotted dies.

The tablets obtained were smooth and uniform, the weight variation is less than 1%, of adequate hardness (4.51 ± 0.37 kgf), with good abrasion resistance, adequate disintegration time and the content of the active principle is found between 90 and 110%.

The statistical treatment of the release profiles of the active principle from the hydrophilic matrices under study was carried out using the Origin 5.0 software with a confidence level of 95%.

Manufacturing formula 2 per tablet:

| Components | Weight (mg) | % |
|---|---|---|
| Active principle | 10 | 10 |
| Hydroxypropylcellulose (HPC) | 30 | 30 |
| Lactose monohydrate | 8 | 8 |
| Starch | 20 | 20 |
| Microcrystalline cellulose | 30 | 30 |
| Silicon dioxide | 1 | 1 |
| Magnesium stearate | 1 | 1 |
| Tablet Weight (mg) | 100 | 100 |

**Example 9: Formula 3 for the manufacture of tablets with EUDRAGIT and lactose of the active principle methyl (2-{[4-(1-naphthylamino)-4-oxobutanoyl]amino}ethyl)dithiocarbamate (3).**

All components were weighed according to the quantities required in the formula. The active principle and the polymeric matrix were sieved through a No. 20 sieve mesh and subsequently mixed for 5 minutes at 30 rpm. Then, every 15 minutes, the rest of the components were added at a speed of 50 rpm, until a homogeneous mixture was obtained. The mixture is then compressed on a high-speed rotary machine, using flat, beveled and unslotted dies.

The tablets obtained were smooth and uniform, the weight variation is less than 1%, adequate hardness (5.01 ± 0.68 kgf), good abrasion resistance, adequate disintegration time and the content of the active principle was between 90 and 110%

The statistical treatment of the release profiles of the active principle from the hydrophilic matrices under study was carried out using the Origin 5.0 software with a confidence level of 95%.

Manufacturing formula 3 per tablet:

| Components | Weight (mg) | % |
|---|---|---|
| Active principle | 10 | 10 |
| EUDRAGIT RS PO | 39 | 39 |
| Lactose monohydrate | 40 | 40 |
| Microcrystalline cellulose | 9 | 9 |
| Silicon dioxide | 1 | 1 |
| Magnesium stearate | 1 | 1 |
| Tablet Weight (mg) | 100 | |

**Example 10: Nasal formulation of methyl (2-{[4-(1-naphthylamino)-4-oxobutanoyl)amino}ethyl)dithiocarbamate (3)**

Solution A: Compound **3** is solubilized in PEG 400, Tocopherol, and glycerol.

Solution B: The HPMC is resuspended in 40% of the water, until its total dissolution with stirring and heat (80 °C).

Solution C: In 60% of water, benzalkonium chloride, $NaH_2PO_4$, $Na_2HPO_4$, NaCl, EDTA, and Tween 80 are dissolved. It is verified that the pH is maintained at 6.3.

Solutions B and C are then combined and stirred until a homogeneous solution is obtained. Then, solution A is slowly added to this solution, stirring and finally, it is filtered through a 0.2 μmol/L membrane.

| Components | Concentration (mg/mL) |
|---|---|
| Active principle **3** | 6 |
| PEG 400 | 3 |
| HPMC | 5 |
| Benzalkonium chloride | 0.2 |
| $NaH_2PO_4$ | 2 |
| $Na_2HPO_4$ | 0.7 |
| NaCl | 7.4 |
| Tween 80 | 0.25 |
| EDTA | 0.15 |
| Glycerol | 5 |
| Water | make up to final volume of 1 mL |

**Example 11: Nasal formulation of 1(N-[4-(1-naphthylamino)-4-oxobutanoyl]-β-alanine (1)**

Solution A: Compound **3** is solubilized in PEG 400, Tocopherol, and glycerol.

Solution B: The HPMC is resuspended in 40% of the water, until its total dissolution with stirring and heat (80 °C).

Solution C: In 60% of water, benzalkonium chloride, $NaH_2PO_4$, $Na_2HPO_4$, NaCl, EDTA, and Tween 80 are dissolved. It is verified that the pH is maintained at 6.3.

Solutions B and C are then combined and stirred until a homogeneous solution is obtained. Then, solution A is slowly added to this solution, stirring and finally, it is filtered through a 0.2 μmol/L membrane.

| Components | Concentration (mg/mL) |
|---|---|
| Active principle **1** | 6 |

(continued)

| Components | Concentration (mg/mL) |
|---|---|
| PEG 400 | 3 |
| CMC | 5 |
| Benzalkonium chloride | 0.2 |
| $NaH_2PO_4$ | 2 |
| $Na_2HPO_4$ | 0.7 |
| NaCl | 7.4 |
| Tocopherol | 0.1 |
| Tween 80 | 0.2 |
| EDTA | 0.1 |
| Glycerol | 5 |
| Water | make up to final volume of 1 mL |

**Example 12. Studies of the dissolution profiles of formulations 1, 2, and 3.**

The dissolution studies took into account the Cuban Standard (CECMED: Validation of Analytical Methods. Regulation No. 41. Havana: CECMED; 2007) and those presented in the United States Pharmacopeia (in USP 36, Rockville: Mack Printing, New York 2013, p 3220- 3221).

The weight of 20 tablets of each batch of Formulation (Examples 7, 8 and 9) was verified. All of them are within the ranges of the weight values of the allowed tablets, for which the drug release profiles of the selected matrix were determined, without error associated with the initial formulation.

Means for the determination of release profiles:

Medium 1, - Simulated gastric fluid without enzymes pH 1.2 ± 0.05

2 g of NaCl (neat for analysis) are weighed out and dissolved in 7 mL of HCl in a 1000 mL flask. The flask is made up to the mark with distilled water. The pH of the solution is 1.2.

Medium 2, - Simulated intestinal fluid without enzyme pH 7.5 ± 0.1

6.8 g of monobasic potassium phosphate was weighed and transferred to a 1000 mL volumetric flask. Later, 250 mL of water was added, the solid was dissolved and 190 mL of 0.2 N NaOH solution and 400 mL of double distilled water were added. Finally, it was made up at 1000 mL with double distilled water. The pH of the solution was adjusted with 0.2 N NaOH to a pH of 7.5 ± 0.1.

Procedure:

Each tablet was placed in the dissolution apparatus (Distek, Evolution 6100 model) with 600 mL of dissolution medium-1 at 37° ÷ 0.5°C, stirred at 30 rpm for 60 minutes. A portion of the test medium was immediately filtered through a 0.45 μmol/L filter at 5, 15, 30 and 60 minutes of testing.

Subsequently, the dissolution medium-1 was drained from the dissolving apparatus and it was replaced by 600 mL of the dissolution medium-2 at a temperature of 37° ± 0.5 °C, stirring at 30 rpm for 8 additional hours. Samples were carried out from 5 to 1080 minutes, the samples were immediately filtered through a 0.45 μmol/L filter, at the indicated times.

The absorbance values of the sample solutions in each dissolution medium were obtained at the maximum absorbance length determined. To quantify the formulated compound, a UV-Vis spectrophotometric calibration curve (Xmax) was performed, in the concentration range of 0.025 to 0.150 mg/mL, and an adjustment blank (dissolution medium) was used.

According to the analysis of the data obtained, the methodology used complies with the parameters of linearity (R> 0.999), precision (reproducibility and repeatability) and accuracy required of an analytical methodology, allowing to

obtain precise and exact results within the concentration range and in the experimental conditions used in this study.

**Example 13. Evaluation of the anticholinergic modulating effect of the compound methyl(2-{[4-(1-naphthylamino)-4-oxobutanoyl]amino}ethyl)dithiocarbamate (3) administered nasally (Formulation according to Example 10) in a model of memory loss induced with chronic use of scopolamine.**

Animals.

Male C57BL / 6 mice (20-25 g), supplied by the National Center for the Laboratory Animals Production (CENPALAB, Cuba) were used. The conditions of adaptation and maintenance of the animals before carrying out the experiment were similar to those developed in non-limiting Example 4.

Experimental design.

At the end of the adaptation period, the animals were randomly distributed according to their body weights in four experimental groups of 10 animals each: two healthy control groups, one administered with a vehicle via the nasal route (G1) and the other with saline solution via. ip (G2); a sick control group, administered with nasal saline and then, with ESC dissolved in saline solution (1.5 mg/kg, ip) (G3) and a treated group, administered with compound **3** prepared according to the formulation of non-limiting Example 10 and after 15 minutes the ESC is administered (1.5 mg/kg, ip) (G4). This experiment was carried out in two experimental series under the same conditions described in order to study the effects of the nasal formulation of compound 3 in two behavioral models: Y-maze in the hidden arm modality and the object discrimination model. In this way, a total of 60 animals were used.

For intranasal administration, the previously described methodology was followed (Hanson, L.R., et al. inJ. Vis. Exp. 2013 (74), e4440, doi: 10.3791 / 4440*)*. Briefly: the mouse head was held in a supine position with the neck extended and subsequently, with the dominant hand, an automatic pipette was loaded with 6 μL of the drug or vehicle. The tip of the pipette was placed near the left nostril and approximately 3 μL of the contents of the pipette were discharged. After 2-3 seconds, the same operation was repeated in the same nostril. At the end of this operation, the animal was kept in the supine position and the same procedure was repeated in this case in the right nostril. Upon completion, the mouse was placed in its cage and after two minutes, another application was made in both nostrils. With this system, the animal was immobilized three times and a total volume of 30 μL was applied, equivalent to a dose of compound 3 of 7.2 mg/kg of weight. 15 minutes after the end of the last administration, each animal was administered i.p. 1.5 mg/kg of ESC or its vehicle (saline solution) and after 30 minutes the behavioral experiments were performed.

Behavioral tests.

Y maze

The study of short-term spatial memory was carried out through the study of novel spatial preference using the Y-maze behavioral paradigm (40 cm long x 9 cm wide x 16 cm high) with extra-labyrinthine tracks around the team, to assess the recognition of spatial memory, dependent on the hippocampus. The test was developed following the same protocol described in non-limiting Example 2. The experiments were carried out between 8 and 12.30 am.

Object discrimination

The Object Recognition Test (ORT) is a commonly used behavioral test for the investigation of various aspects of learning and memory, particularly recognition memory, in rodent models of CNS disorders. The protocol used consists of three phases: a first habituation phase (day 1), a second familiarization phase (day 2) and finally the test phase (day 3). All phases lasted five minutes and were carried out on three consecutive days. In the first phase, the animals were placed inside a plexiglass box (25 cm x 25 cm x 25 cm). The behavior of the animal was recorded using a video camera. This first day the animals were brought to the test room 30 minutes before the experiment to become familiar with the environment. The mice were then allowed to freely explore the box in the absence of objects for five minutes. On the second day each mouse underwent the training test in which two identical objects were placed in two opposite positions inside the box at the same time, same distance from the nearest corner. During this phase, the mice were allowed to explore the identical objects for five minutes and then returned to their home cages. During the test phase (day 3) the animals were placed back in the same box, where one of the two familiar objects was exchanged for a new one, before this phase. All the objects used in this study were different in shape and color but identical in size. They were fixed to the floor of the box to prevent movement. To avoid the existence of olfactory signals, the entire box and objects were always cleaned with 70% alcohol, after each test. The ability to discriminate the new object from the relative was observed

through the time necessary to explore the objects. The object exploration time was defined as the period of time during which the animal directed its nose at a distance of 2 cm from the object, or smelled or kicked it. Sitting or standing on the object was not recognized as exploration. The scan time was manually analyzed using smart video software. The discrimination index (ID) was calculated as follows.

$$ID = \text{novel object exploration time / novel object exploration time + familiar object exploration time.}$$

Statistical analysis.

All data are expressed as the mean ± standard error of the mean (SEM), and were analyzed using a one-way analysis of variance followed by Turkey's *post-hoc* test. A p <0.05 was considered a statistically significant difference. Statistical analysis was performed using GraphPadPrism version 5 (GraphPad Software, Inc., San Diego, USA).

## Claims

1. A pharmaceutical composition of a compound that acts as a multi-target target therapeutic agent for the treatment of Alzheimer's disease, **characterized in that** compound is selected from the group of compounds with Formula I

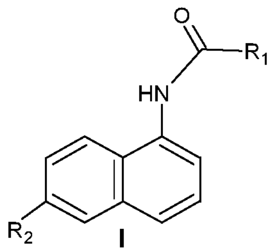

**I**

where:

$R_1$: -alkylenyl-C (O) NH-alkylenyl-$R_3$, -alkylenyl-C (O) O-$R_4$;
$R_3$: -COOH, -OH, -SH, -$NH_2$, -NH-alkyl-, -NH-alkylenyl-$NH_2$, -NH-alkylenyl-NH-C (O) -alkylenyl-S-$R_5$, -NH-dithiocarbamate- alkyl, -N-alkyl-dithiocarbamate alkaline earth metal salts; or salts of the groups mentioned above, pharmaceutically acceptable.
$R_4$: succinimidyl group
$R_5$: -H, -C (O) -alkyl, -C (O) -$C_6H_5$; and
$R_2$: -H, -alkyl.
The term "alkyl" is **characterized by** being a linear or branched aliphatic chain of saturated carbon atoms and hydrogen atoms, preferably methyl or ethyl. The term "alkylenyl" refers to a divalent analog of a linear or branched alkyl group, preferably methyleneyl (-$CH_2$-), ethylenyl (-$CH_2CH_2$-), or propylenyl (-$CH_2CH_2CH_2$-).
where the pharmaceutical composition contains pharmaceutically acceptable excipients,
where these compounds, their salts, hydrates, enantiomers, isomers, metabolites, prodrugs cross the blood-brain barrier of the brain of a living mammal.

2. The pharmaceutical composition of a compound that acts as a multi-target therapeutic agent of claim 1, **characterized in** the composition interacts with brain cholinergic cells, inhibits the pathological activity of acetylcholinesterase and restores the physiological levels of acetylcholine.

3. The pharmaceutical composition of a compound that acts as a multi-target therapeutic agent of claim 1, **characterized in** the composition interacts with brain glutamatergic cells, inhibits pathological activation of NMDA and kainate receptors and restores the physiological functioning of the glutamatergic system.

4. The pharmaceutical composition of a compound that acts as a multi-target therapeutic agent of claim 1, **characterized in** the composition interacts with the cerebral mitochondria, inhibits the dissipation of the mitochondrial membrane

potential and prevents swelling to maintain the physiological levels of reactive oxygen species.

5. The pharmaceutical composition of a compound that acts as a multi-target therapeutic agent of claim 1, **characterized in** the composition is administered orally, topically, systemically, intravenously, subcutaneously, intraperitoneally, intramuscularly and nasal, or combinations thereof

6. The pharmaceutical composition of a compound that acts as a multi-target therapeutic agent of claims 1 and 5, **characterized in** the oral formulation of the pharmaceutical composition in the form of tablets contains from 1 mg to 100 mg of the active principle.

7. The pharmaceutical composition of a compound that acts as a multi-target therapeutic agent of claims 1 and 6, **characterized in** the tablets of the pharmaceutical composition contain a polymeric matrix, which is selected from the group: polyethylene oxide, hydroxypropylmethylcellulose (HPMC), hydroxypropylcellulose (HPC) ), sodium carboxymethyl cellulose (CCNa), cellulose derivatives, Eudragit RS PO and polyvinylpyrrolidone, or combinations thereof.

8. The pharmaceutical composition of a compound that acts as a multi-target therapeutic agent of claim 7, **characterized in** the matrix of the tablets of the pharmaceutical composition is in a proportion of 5% w/w to 80% w/w, preferably from 10 % w/w to 70% w/w.

9. The pharmaceutical composition of a compound that acts as a multi-target therapeutic agent of claims 1 and 5, **characterized in** the nasal formulation of the pharmaceutical composition contains 0.1 mg to 25 mg of the active principle per dose.

10. The pharmaceutical composition of a compound that acts as a multi-target therapeutic agent of claims 1 and 9, **characterized in** the nasal formulation of the pharmaceutical composition contains bioadhesive excipients, in a proportion from 0.05 to 6%, selected from the group: hydroxypropylmethylcellulose (HMPC) , hydroxypropyl cellulose (HPC), methyl cellulose (MC), carboxymethyl cellulose (CMC) and polyacrylic acid (carbol) derivatives, or combinations thereof.

11. The pharmaceutical composition of a compound that acts as a multi-target therapeutic agent of claims 1 and 10, **characterized in** the nasal formulation of the pharmaceutical composition has a viscosity of 10 to 60 mPas.

12. The pharmaceutical composition of a compound that acts as a multi-target therapeutic agent of claims 1, 9, 10 and 11, **characterized in** the nasal formulation of the pharmaceutical composition contains PEG at a concentration of 0.09 to 1.8%; tocopherol at a concentration of 0.08 to 1%; an antimicrobial preservative selected from the group: benzalkonium chloride at a concentration of 0.007 to 0.47%, disodium EDTA at a concentration of 0.003 to 0.19% and propyl paraben at a concentration of 0.005 to 0.04%, or combinations thereof; and a humectant selected from the group: glycerol at a concentration of 3.5 to 5.5% and Tween 80 at a concentration of 0.17 to 0.38%; or combinations of them.

13. The pharmaceutical composition of a compound that acts as a multi-target therapeutic agent of claims 1 and 12, **characterized in** the nasal formulation of the pharmaceutical composition has a pH between 5.5 and 6.5, preferably 6.3.

14. A method for the monotherapy treatment of Alzheimer’s disease, **characterized in** the method comprises:

a) the administration of a pharmaceutical composition of a compound with multi-target action of Formula I,

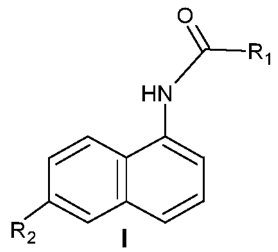

I

where:

$R_1$: -alkylenyl-C (O) NH-alkylenyl-$R_3$, -alkylenyl-C (O) O-$R_4$;
$R_3$: -COOH, -OH, -SH, -$NH_2$, -NH-alkyl-, -NH-alkylenyl-$NH_2$, -NH-alkylenyl-NH-C (O) -alkylenyl-S-$R_5$, -NH-dithiocarbamate- alkyl, -N-alkyl-dithiocarbamate alkaline earth metal salts; or salts of the groups mentioned above, pharmaceutically acceptable.
$R_4$: succinimidyl group;
$R_5$: -H, -C (O) -alkyl, -C (O) -$C_6H_5$; and
$R_2$: -H, -alkyl.
The term "alkyl" is **characterized by** being a linear or branched aliphatic chain of saturated carbon atoms and hydrogen atoms, preferably methyl or ethyl. The term "alkylenyl" refers to a divalent analog of a linear or branched alkyl group, preferably methyleneyl (-$CH_2$-), ethylenyl (-$CH_2CH_2$-), or propylenyl (-$CH_2CH_2CH_2$-).
where the pharmaceutical composition contains pharmaceutically acceptable excipients,
where the pharmaceutical composition is administered by oral, topical, systemic, intravenous, subcutaneous, intraperitoneal, intramuscular and nasal routes, or combinations thereof,

b) the pharmaceutical composition of the compound of step a) crosses the blood-brain barrier of the brain of a living mammal,
c) contacting the brain cholinergic cells with the compound of the pharmaceutical composition of step b) and inhibiting the pathological activity of acetylcholinesterase to restore the physiological level of acetylcholine,
d) contacting the brain glutamatergic cells with the compound of the pharmaceutical composition of step b) and inhibiting the pathological activation of the NMDA and kainate receptors to restore the physiological functioning of the glutamatergic system, and
e) contacting the brain mitochondria with a compound of the pharmaceutical composition of step b) to inhibit the dissipation of the mitochondrial membrane potential and prevent swelling to maintain physiological levels of reactive oxygen species.

**15.** The method for the monotherapy treatment of Alzheimer's disease of claim 14, characterized that the oral pharmaceutical composition in the form of tablets contains from 1 mg to 100 mg of the active principle.

**16.** The method for the monotherapy treatment of Alzheimer's Disease of claims 14 and 15, **characterized in** the tablets contain a polymeric matrix, which is selected from the group: polyethylene oxide, hydroxypropylmethylcellulose (HPMC), hydroxypropylcellulose (HPC), sodium carboxymethyl cellulose (CCNa), cellulose derivatives, Eudragit RS PO, polyvinylpyrrolidone or combinations thereof.

**17.** The method for the monotherapy treatment of Alzheimer's Disease of claims 14 and 16, characterized that the matrix of the tablets is in a proportion of 5% w/w to 80% w/w, preferably from 10 % w/w to 70% w/w.

**18.** The method for the monotherapy treatment of Alzheimer's Disease of claims 14 and 15, characterized that the tablets use excipients selected from the group: starches, lactose, sucrose, sorbitol, mannitol and other sugars, talc, colloidal silicon dioxide , carbonates, magnesium oxides, calcium phosphates, titanium dioxide, povidones, gelatin, lacto-proteins, citrates, tartrates, alginates, dextran, silicone elastomers, polysorbates, amylopectin, parabens, animal and vegetable oils, propylene glycol, sterile water, mono or polyhydric alcohols, magnesium stearate, calcium stearate, sodium stearyl fumarate, sodium lauryl sulfate, and glycerin, or combinations thereof.

**19.** The method for the monotherapy treatment of Alzheimer's disease of claim 14, characterized that the nasal pharmaceutical composition contains 0.1 mg to 25 mg of the active principle.

**20.** The method for the monotherapy treatment of Alzheimer's disease of claims 14 and 19, characterized that the nasal formulation contains bioadhesive excipients, in a proportion from 0.05 to 6%, selected from the group: hydroxypropylmethylcellulose (HMPC), hydroxypropylcellulose (HPC), methylcellulose (MC), carboxymethylcellulose (CMC) and polyacrylic acid (carbol) derivatives, or combinations thereof.

**21.** The method for the monotherapy treatment of Alzheimer's disease of claims 14 and 20, characterized that the nasal formulation has a viscosity of 10 to 60 mPas.

**22.** The method for the monotherapy treatment of Alzheimer's Disease of claims 14 and 19, characterized that the nasal

formulation contains PEG, tocopherol, an antimicrobial preservative selected from the group: benzalkonium chloride, disodium EDTA, methyl paraben and propyl paraben, or combinations between them, and a humectant selected from the group: glycerol and Tween 80 or combinations thereof.

**23.** The method for the monotherapy treatment of Alzheimer's disease of claims 1 and 22, characterized that the nasal formulation has a pH between 6 and 7.5, preferably 6.3.

**24.** A pharmaceutical composition of a compound that acts as a multi-target therapeutic agent for use in the treatment of Alzheimer's Disease, characterized that the compound is selected from the group of compounds with Formula I

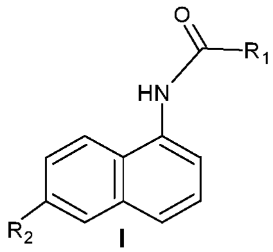

**I**

where:

$R_1$: -alkylenyl-C (O) NH-alkylenyl-$R_3$, -alkylenyl-C (O) O-$R_4$;
$R_3$: -COOH, -OH, -SH, -$NH_2$, -NH-alkyl-, -NH-alkylenyl-$NH_2$, -NH-alkylenyl-NH-C (O) -alkylenyl-S-$R_5$, -NH-dithiocarbamate- alkyl, -N-alkyl-dithiocarbamate alkaline earth metal salts; or salts of the groups mentioned above, pharmaceutically acceptable.
$R_4$: succinimidyl group;
$R_5$: -H, -C (O) -alkyl, -C (O) -$C_6H_5$; and
$R_2$: -H, -alkyl.
The term "alkyl" is **characterized by** being a linear or branched aliphatic chain of saturated carbon atoms and hydrogen atoms, preferably methyl or ethyl. The term "alkylenyl" refers to a divalent analog of a linear or branched alkyl group, preferably methyleneyl (-$CH_2$-), ethylenyl (-$CH_2CH_2$-), or propylenyl (-$CH_2CH_2CH_2$),
where the pharmaceutical composition contains pharmaceutically acceptable excipients,
where these compounds, their salts, hydrates, enantiomers, isomers, metabolites, prodrugs cross the blood-brain barrier of the brain of a living mammal.

**25.** The pharmaceutical composition of a compound that acts as a multi-target therapeutic agent for use in the treatment of Alzheimer's disease of claim 24, characterized that the composition interacts with brain cholinergic cells, inhibits the pathological activity of acetylcholinesterase and restores levels Physiological effects of acetylcholine.

**26.** The pharmaceutical composition of a compound that acts as a multi-target therapeutic agent for use in the treatment of Alzheimer's disease of claim 24, characterized that the composition interacts with brain glutamatergic cells, inhibits pathological activation of NMDA and kainate receptors and restores the physiological functioning of the glutamatergic system.

**27.** The pharmaceutical composition of a compound that acts as a multi-target therapeutic agent for use in the treatment of Alzheimer's disease of claim 24, characterized that the composition interacts with brain mitochondria, inhibits the dissipation of the mitochondrial membrane potential and prevents swelling to maintain physiological levels of reactive oxygen species.

**28.** The pharmaceutical composition of a compound that acts as a multi-target therapeutic agent for use in the treatment of Alzheimer's Disease of claim 24, characterized that the composition is administered orally, topically, systemically, intravenously, subcutaneously, intraperitoneally, intramuscularly, nasal or combinations of them.

**29.** The pharmaceutical composition of a compound that acts as a multi-target therapeutic agent for use in the treatment of Alzheimer's disease of claims 24 and 28, characterized that the oral pharmaceutical composition in the form of tablets contains from 1 mg to 100 mg of the active principle.

**30.** The pharmaceutical composition of a compound that acts as a multi-target therapeutic agent for use in the treatment of Alzheimer's Disease of claims 24 and 29, characterized that the pharmaceutical composition, the tablets contain a polymeric matrix, which is selected from the group: polyethylene oxide, hydroxypropylmethylcellulose (HPMC), hydroxypropylcellulose (HPC), sodium carboxymethyl cellulose (CCNa), cellulose derivatives, Eudragit RS PO and polyvinylpyrrolidone, or combinations thereof.

**31.** The pharmaceutical composition of a compound that acts as a multi-target therapeutic agent for use in the treatment of Alzheimer's Disease of claim 30, characterized that the pharmaceutical composition the matrix of the tablets is in a proportion of 5% w/pa 80% w/w, preferably from 10% w/w to 70% w/w.

**32.** The pharmaceutical composition of a compound that acts as a multi-target therapeutic agent for use in the treatment of Alzheimer's disease of claims 24 and 28, characterized that the nasal pharmaceutical composition contains 0.1 mg to 25 mg of the active principle per dose.

**33.** The pharmaceutical composition of a compound that acts as a multi-target therapeutic agent for use in the treatment of Alzheimer's disease of claims 24 and 32, characterized that the nasal formulation contains bioadhesive excipients, in a proportion from 0.05 to 6%, selected from the group: hydroxypropylmethylcellulose (HMPC), hydroxypropylcellulose (HPC), methylcellulose (MC), carboxymethylcellulose (CMC) and derivatives of polyacrylic acid (carbol), or combinations thereof.

**34.** The pharmaceutical composition of a compound that acts as a multi-target therapeutic agent for use in the treatment of Alzheimer's Disease of claims 24 and 33, characterized that the nasal formulation has a viscosity of 10 to 60 mPas.

**35.** The pharmaceutical composition of a compound that acts as a multi-target therapeutic agent to be used in the treatment of Alzheimer's Disease of claims 24, 32 and 34, characterized that the nasal formulation contains PEG at a concentration of 0.09 to 1.8%; tocopherol at a concentration of 0.08 to 1%; an antimicrobial preservative selected from the group: benzalkonium chloride at a concentration of 0.007 to 0.47%, disodium EDTA at a concentration of 0.003 to 0.19% and propyl paraben at a concentration of 0.005 to 0.04%, or combinations thereof; and a humectant selected from the group: glycerol at a concentration of 3.5 to 5.5% and Tween 80 at a concentration of 0.17 to 0.38%; or combinations of them.

**36.** The pharmaceutical composition of a compound that acts as a multi-target therapeutic agent for use in the treatment of Alzheimer's Disease of claims 24 and 35, characterized that the nasal formulation has a pH between 5.5 and 6.5, preferably 6.3.

| Compound | Structure | Physico-chemical properties |
|---|---|---|
| N-[4-(1-naphthylamino)-4-oxobutanoyl]-β-alanine (**1**) | 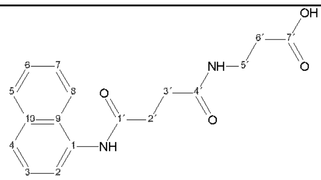 | MOLECULAR MASS: 314.3 g/mol. White, crystalline powder. QUALITY SPECIFICATIONS. HR-TOF/MS (m/z): calcd. for $C_{17}H_{18}N_2O_4$: 314.33; found 315.13 (M + H) +. T.f: 172.5-174.8 ° C. Rf: 0.1 (ethyl acetate). HPLC. Phenomenex C18 chromatographic column (150 x 4.6 /mmol/L, 4 /µmol/L, 80Å). Mobile Phase: $H_2O$/TFA (0.1%) ACN/TFA (0.05%). Retention time: 25.051 minutes Purity: 99.76%. |
| Acid 6-{[4-(1-naphthylamino)-4-oxobutanoyl]amino} hexanoic acid (**2**): | 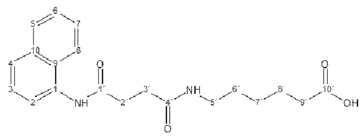 | MOLECULAR MASS: 356.3 g/mol. White, crystalline powder. QUALITY SPECIFICATIONS. HR-TOF/MS (m/z): calcd. for $C_{20}H_{24}N_2O_4$: 357.42 (M * H) *; found 357.18. T.f: 129.9-133 ° C. Rf: 0.6 ($CHCl_3$: $CH_3OH$, 10: 3). HPLC. Phenomenex C18 chromatographic column (150 x 4.6 /mmol/L, 4 /µmol/L, 80Å). Mobile Phase: $H_2O$/TFA (0.1%) ACN/TFA (0.05%). Retention time: 27.935 minutes Purity: 99.19%. |
| Methyl (2-{[4-(1-naphthylamino) -4-oxobutanoyl]amino} ethyl) dithiocarbamate (**3**): | 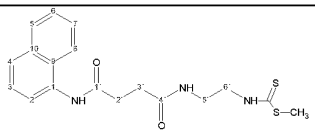 | MOLECULAR MASS: 375.5 g/mol. Light yellow, crystalline powder. QUALITY SPECIFICATIONS. HR-TOF/MS (m/z): calcd. for $C_{18}H_{21}N_3O_2S_2$: 375,511; found 377.121 (M + D) +. T.f: 140-141 ° C. Rf: 0.3 (ethyl acetate). HPLC. Phenomenex C18 chromatographic column (150 x 4.6 /mmol/L, 4 /µmol/L, 80Å). Mobile Phase: $H_2O$/TFA (0.1%) ACN/TFA (0.05%). Retention time: 31.428 minutes Purity: 96.59%. |

Figure 1

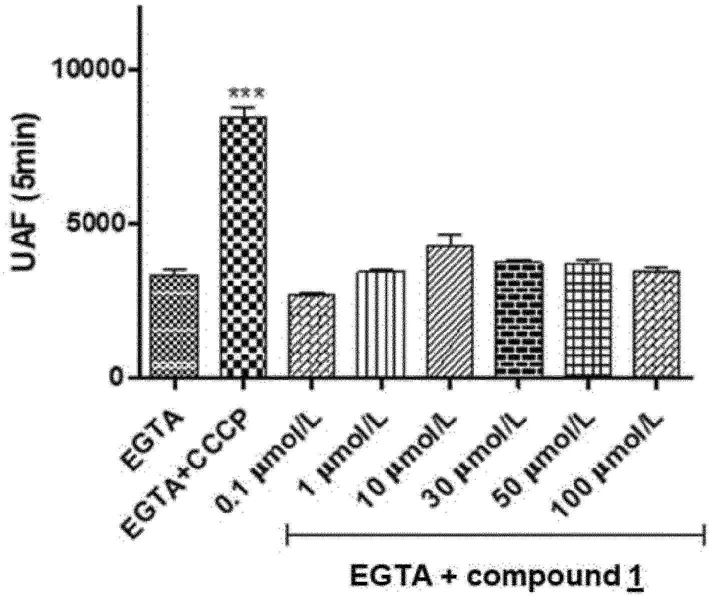
10000
5000
0
UAF (5min)
***
EGTA
EGTA+CCCP
0.1 µmol/L
1 µmol/L
10 µmol/L
30 µmol/L
50 µmol/L
100 µmol/L
EGTA + compound 1

Figure 2

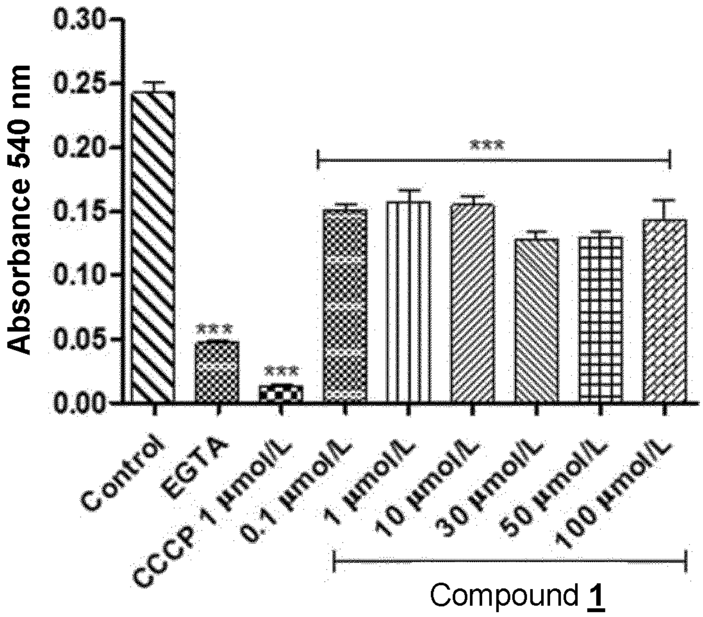
Absorbance 540 nm
0.30
0.25
0.20
0.15
0.10
0.05
0.00
***
***
***
Control
EGTA
CCCP 1 µmol/L
0.1 µmol/L
1 µmol/L
10 µmol/L
30 µmol/L
50 µmol/L
100 µmol/L
Compound 1

Figure 3

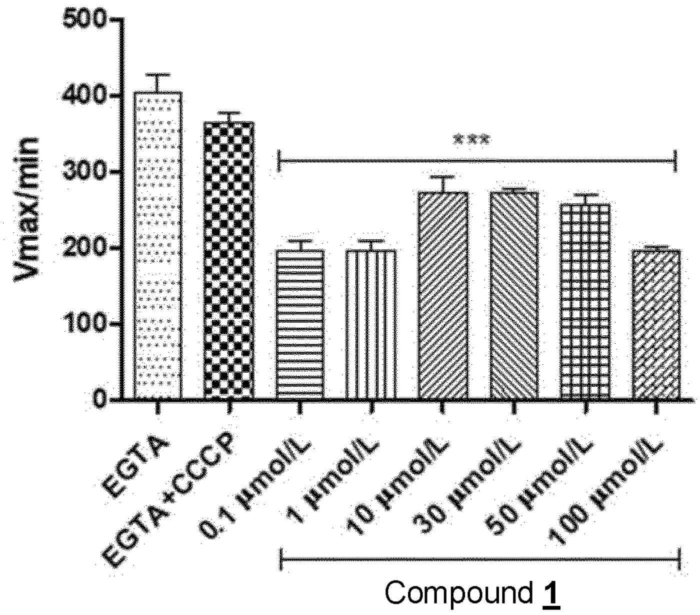

Vmax/min
500
400
300
200
100
0
***
EGTA
EGTA+CCCP
0.1 µmol/L
1 µmol/L
10 µmol/L
30 µmol/L
50 µmol/L
100 µmol/L
Compound 1


Figure 4

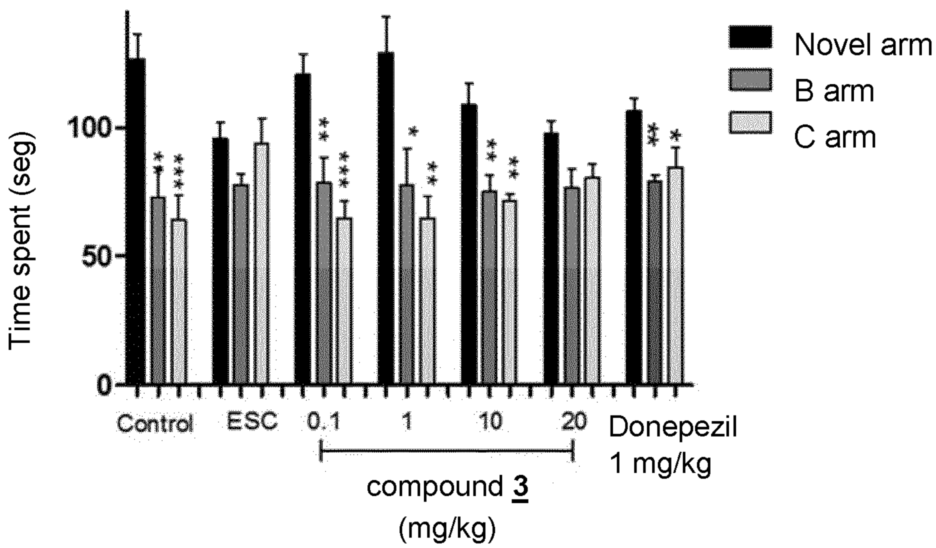
Time spent (seg)
100
50
0
Control
ESC
0.1
1
10
20
Donepezil
1 mg/kg
compound 3
(mg/kg)
Novel arm
B arm
C arm

Figure 5

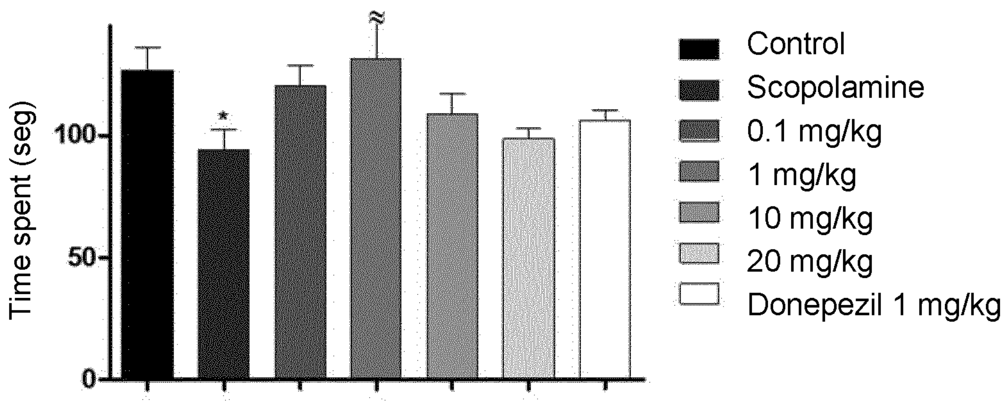
Time spent (seg)
100
50
0
*
≈
Control
Scopolamine
0.1 mg/kg
1 mg/kg
10 mg/kg
20 mg/kg
Donepezil 1 mg/kg

Figure 6

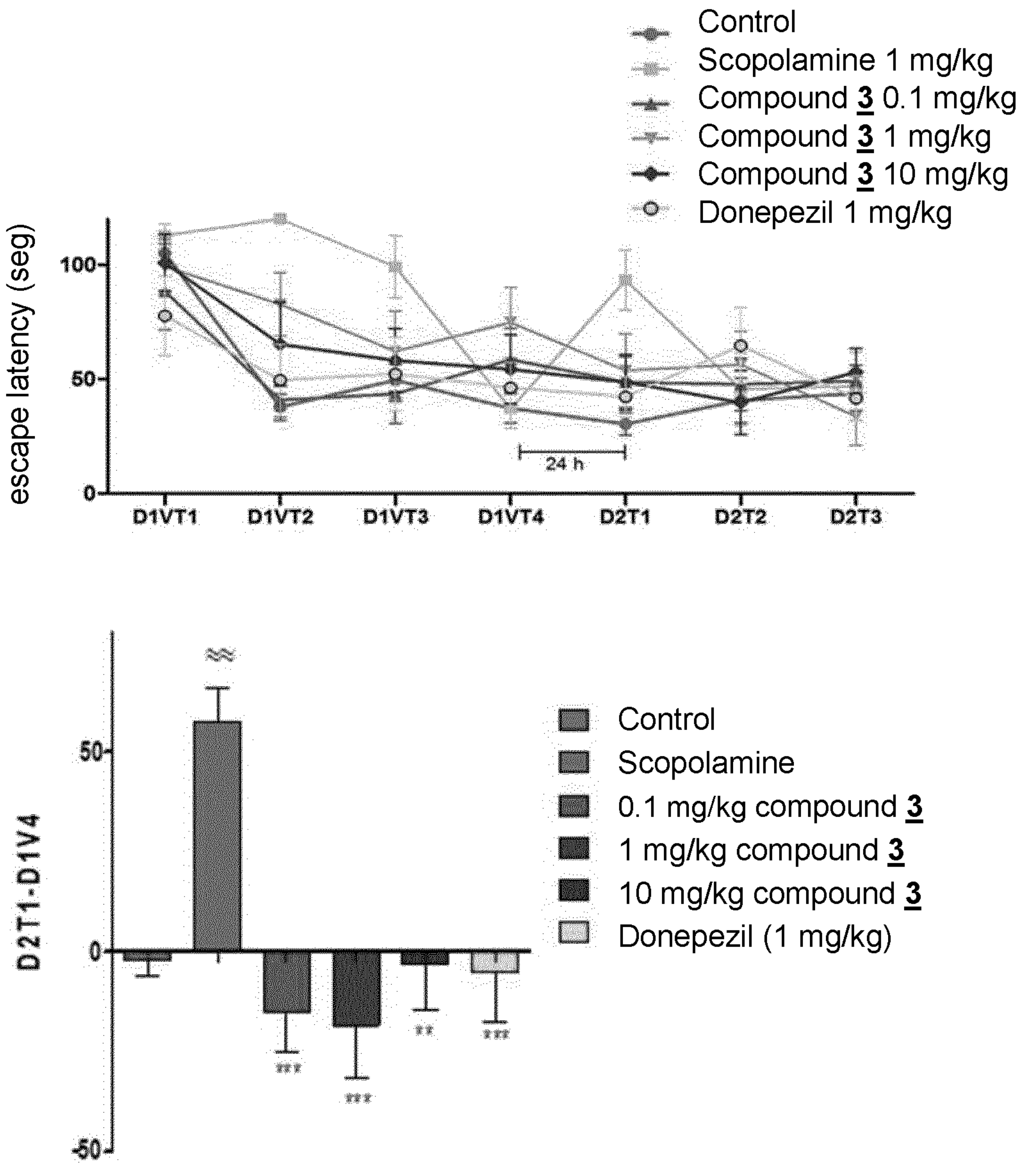
Control
Scopolamine 1 mg/kg
Compound 3 0.1 mg/kg
Compound 3 1 mg/kg
Compound 3 10 mg/kg
Donepezil 1 mg/kg
escape latency (seg)
100
50
0
24 h
D1VT1
D1VT2
D1VT3
D1VT4
D2T1
D2T2
D2T3
Control
Scopolamine
0.1 mg/kg compound 3
1 mg/kg compound 3
10 mg/kg compound 3
Donepezil (1 mg/kg)
D2T1-D1V4
50
0
-50
***
***
**
***

Figure 7

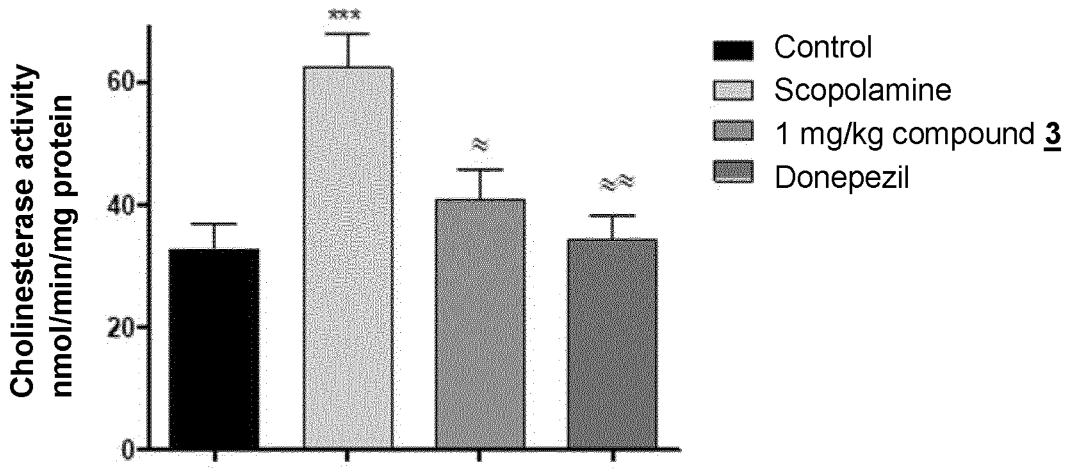
Cholinesterase activity
nmol/min/mg protein
0
20
40
60
***
≈
≈≈
Control
Scopolamine
1 mg/kg compound 3
Donepezil

Figure 8

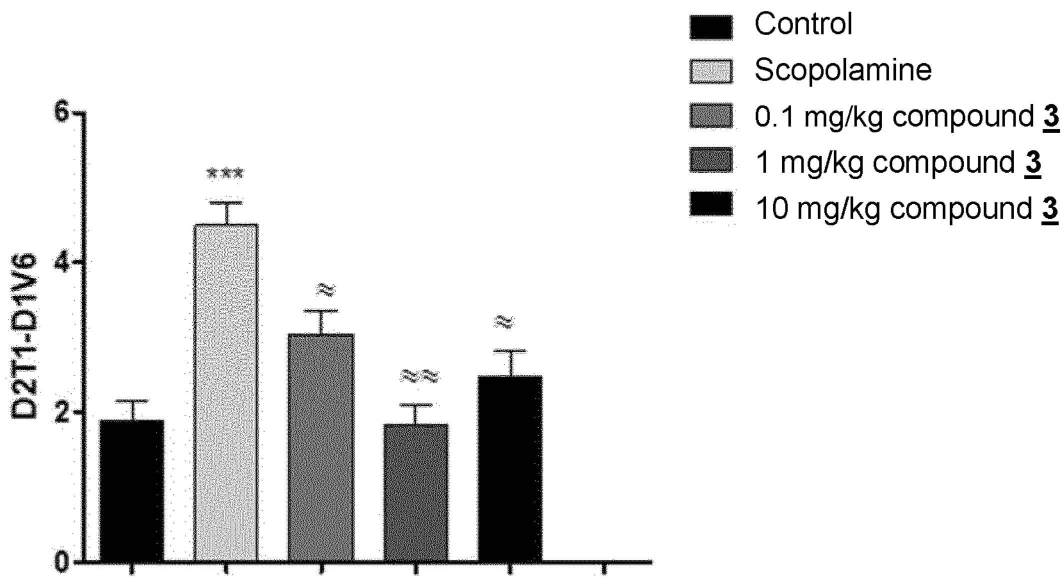
Control
Scopolamine
0.1 mg/kg compound 3
1 mg/kg compound 3
10 mg/kg compound 3
D2T1-D1V6
6
4
2
0
***
≈
≈≈
≈

Figure 9

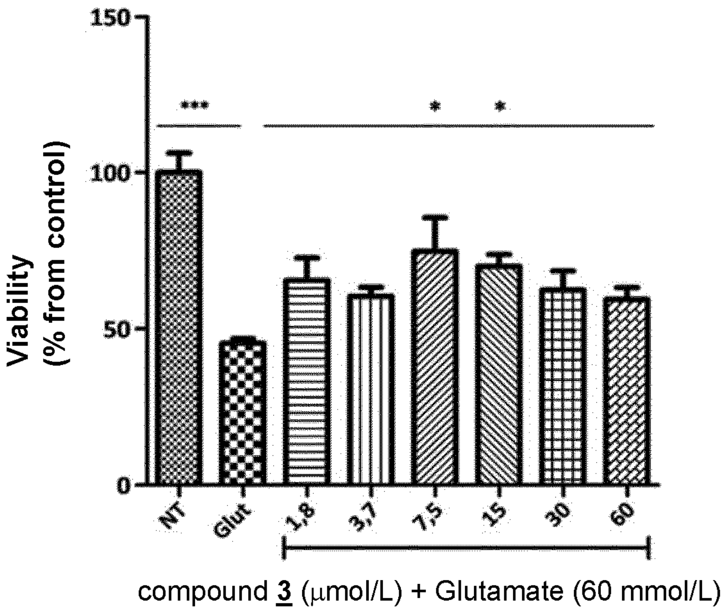
150
100
50
0
Viability
(% from control)
***
*
*
NT
Glut
1,8
3,7
7,5
15
30
60
compound 3 (μmol/L) + Glutamate (60 mmol/L)

Figure 10

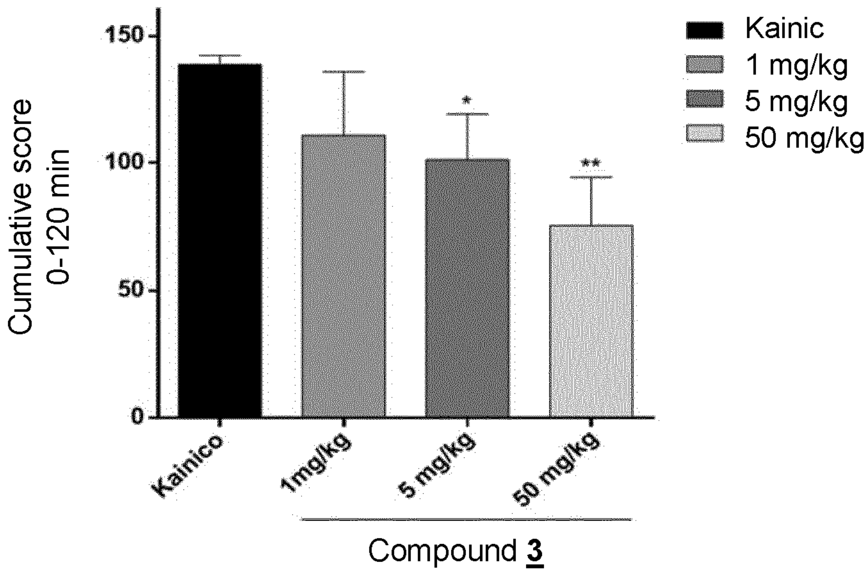
Cumulative score
0-120 min
150
100
50
0
Kainico
1mg/kg
5 mg/kg
50 mg/kg
Compound 3
Kainic
1 mg/kg
5 mg/kg
50 mg/kg
*
**

Figure 11

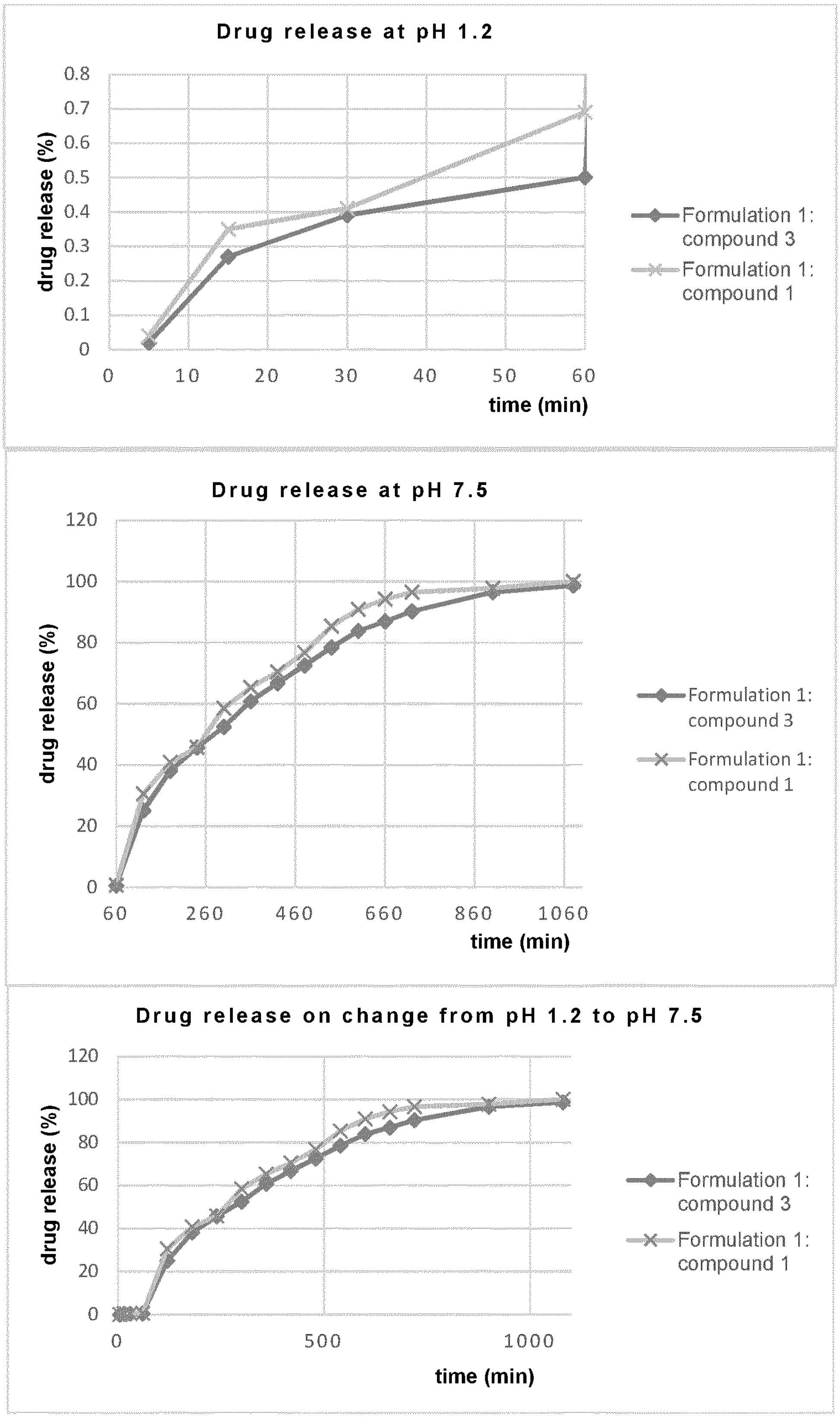
Drug release at pH 1.2
drug release (%)
0.8
0.7
0.6
0.5
0.4
0.3
0.2
0.1
0
0
10
20
30
40
50
60
time (min)
Formulation 1: compound 3
Formulation 1: compound 1
Drug release at pH 7.5
drug release (%)
120
100
80
60
40
20
0
60
260
460
660
860
1060
time (min)
Formulation 1: compound 3
Formulation 1: compound 1
Drug release on change from pH 1.2 to pH 7.5
drug release (%)
120
100
80
60
40
20
0
0
500
1000
time (min)
Formulation 1: compound 3
Formulation 1: compound 1

Figure 12

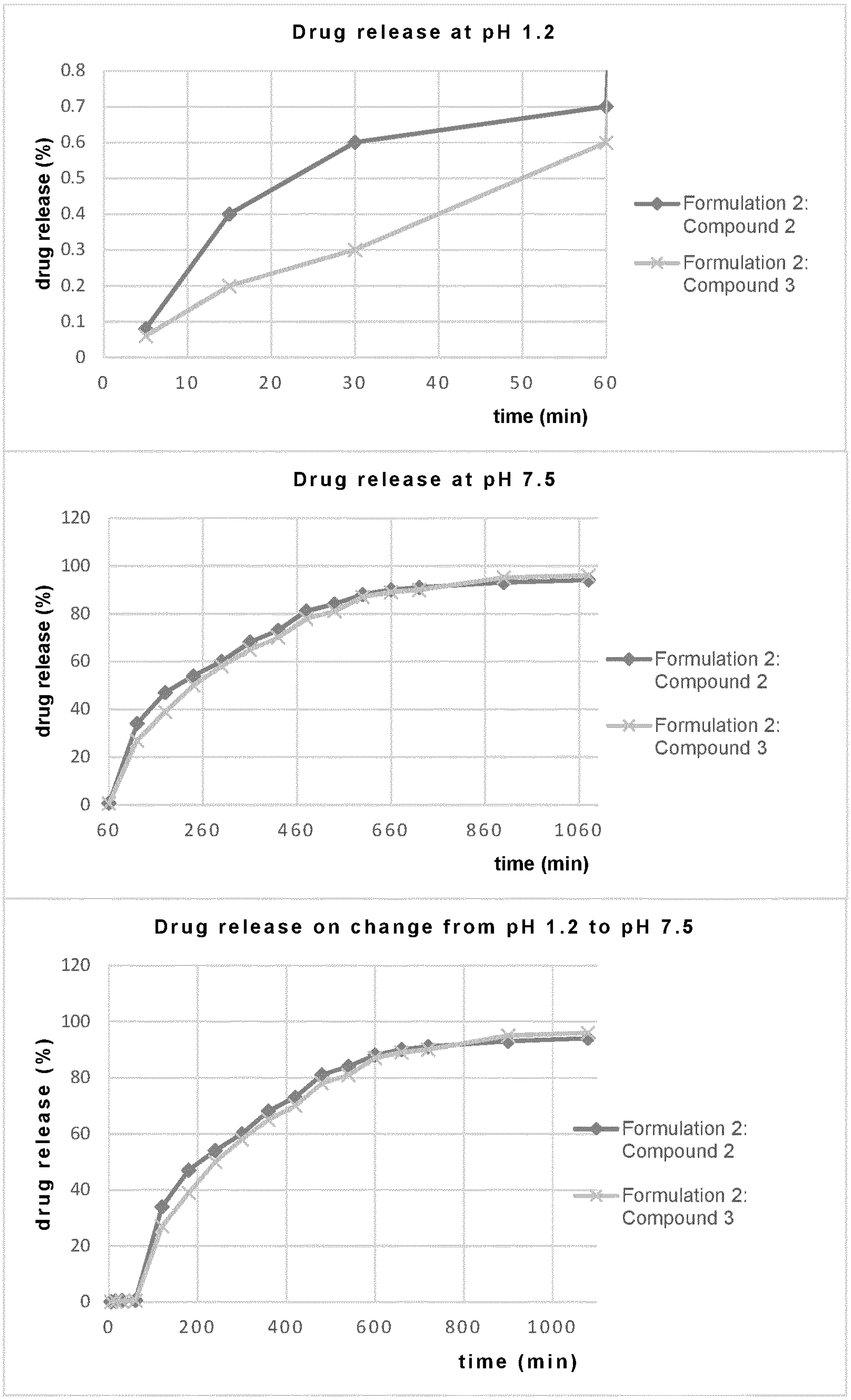
Drug release at pH 1.2
drug release (%)
0.8
0.7
0.6
0.5
0.4
0.3
0.2
0.1
0
0
10
20
30
40
50
60
time (min)
Formulation 2: Compound 2
Formulation 2: Compound 3
Drug release at pH 7.5
drug release (%)
120
100
80
60
40
20
0
60
260
460
660
860
1060
time (min)
Formulation 2: Compound 2
Formulation 2: Compound 3
Drug release on change from pH 1.2 to pH 7.5
drug release (%)
120
100
80
60
40
20
0
0
200
400
600
800
1000
time (min)
Formulation 2: Compound 2
Formulation 2: Compound 3

Figure 13

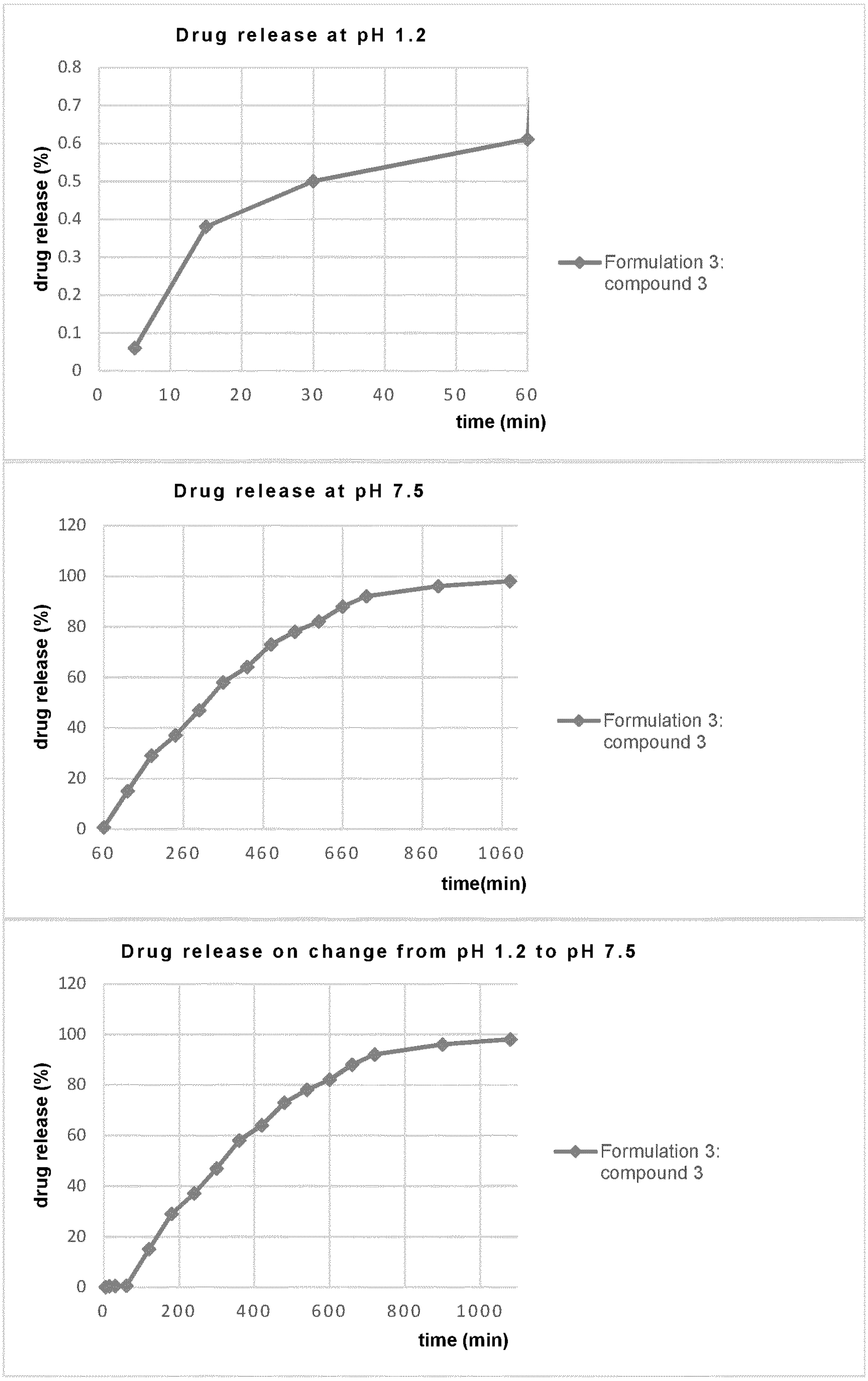
Drug release at pH 1.2
drug release (%)
0.8
0.7
0.6
0.5
0.4
0.3
0.2
0.1
0
0
10
20
30
40
50
60
time (min)
Formulation 3: compound 3
Drug release at pH 7.5
drug release (%)
120
100
80
60
40
20
0
60
260
460
660
860
1060
time(min)
Formulation 3: compound 3
Drug release on change from pH 1.2 to pH 7.5
drug release (%)
120
100
80
60
40
20
0
0
200
400
600
800
1000
time (min)
Formulation 3: compound 3

Figure 14

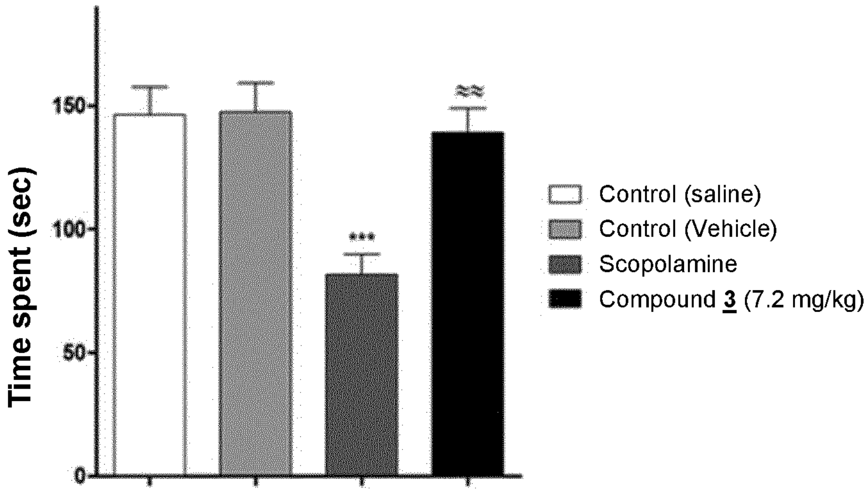
Time spent (sec)
150
100
50
0
***
≈≈
Control (saline)
Control (Vehicle)
Scopolamine
Compound 3 (7.2 mg/kg)

Figure 15

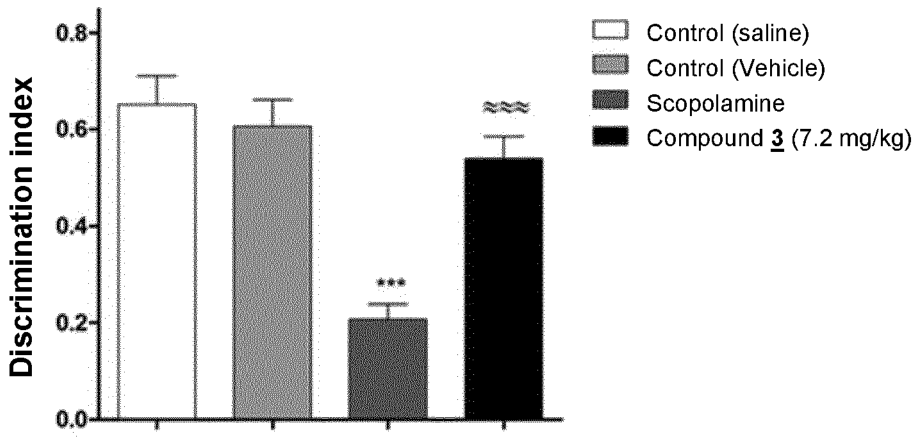
Discrimination index
0.8
0.6
0.4
0.2
0.0
***
≈≈≈
Control (saline)
Control (Vehicle)
Scopolamine
Compound 3 (7.2 mg/kg)

Figure 16

# INTERNATIONAL SEARCH REPORT

International application No
PCT/CU2021/050012

**A. CLASSIFICATION OF SUBJECT MATTER**
INV. A61K31/167 A61K31/27 A61P25/28 A61K9/00 A61K9/20 A61K47/06
ADD.

According to International Patent Classification (IPC) or to both national classification and IPC

**B. FIELDS SEARCHED**

Minimum documentation searched (classification system followed by classification symbols)
A61K A61P

Documentation searched other than minimum documentation to the extent that such documents are included in the fields searched

Electronic data base consulted during the international search (name of data base and, where practicable, search terms used)

EPO-Internal, CHEM ABS Data, WPI Data

**C. DOCUMENTS CONSIDERED TO BE RELEVANT**

| Category* | Citation of document, with indication, where appropriate, of the relevant passages | Relevant to claim No. |
|---|---|---|
| X | US 2016/106691 A1 (CARRAZANA MARQUIZA SABLON [CU] ET AL) 21 April 2016 (2016-04-21) | 1-36 |
| Y | claims 1-7<br>paragraphs [0046], [0055]<br>figures 1-15; examples 1-8; compounds A-B, D-E, G | 1-5,7,8, 10,13, 14, 16-18, 20, 23-28, 30,31, 33,36 |
| | ----- | |

-/--

[x] Further documents are listed in the continuation of Box C.

[x] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance;; the claimed invention cannot be considered novel or cannot be considered to involve an inventive step when the document is taken alone

"Y" document of particular relevance;; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 16 March 2022 | 25/03/2022 |

| Name and mailing address of the ISA/ | Authorized officer |
|---|---|
| European Patent Office, P.B. 5818 Patentlaan 2<br>NL - 2280 HV Rijswijk<br>Tel. (+31-70) 340-2040,<br>Fax: (+31-70) 340-3016 | Renard, Delphine |

Form PCT/ISA/210 (second sheet) (April 2005)

page 1 of 2

## INTERNATIONAL SEARCH REPORT

International application No
PCT/CU2021/050012

C(Continuation). DOCUMENTS CONSIDERED TO BE RELEVANT

| Category* | Citation of document, with indication, where appropriate, of the relevant passages | Relevant to claim No. |
|---|---|---|
| X | RIVERA-MARRERO SUCHITIL ET AL: "A new naphthalene derivative with anti-amyloidogenic activity as potential therapeutic agent for Alzheimer's disease", BIOORGANIC, ELSEVIER, AMSTERDAM, NL, vol. 28, no. 20, 11 August 2020 (2020-08-11), XP086296996, ISSN: 0968-0896, DOI: 10.1016/J.BMC.2020.115700 [retrieved on 2020-08-11] | 1-36 |
| Y | whole document, in particular the administration of compound 8 to a mice model of Alzheimer | 1-5,7,8, 10,13, 14, 16-18, 20, 23-28, 30,31, 33,36 |
| | ----- | |
| X | WO 2020/094161 A1 (CENT NEUROCIENCIAS CUBA) 14 May 2020 (2020-05-14) | 1-13 |
| Y | claims 1-11<br>paragraph [0012] | 1-5,7,8, 10,13, 14, 16-18, 20, 23-28, 30,31, 33,36 |
| | ----- | |
| X,P | EP 3 878 475 A1 (CENTRO DE NEUROCIENCIAS DE CUBA [CU] ET AL.) 15 September 2021 (2021-09-15) | 1-13 |
| Y,P | claims 1-14<br>paragraph [0011]<br>example 8 | 1-5,7,8, 10,13, 14, 16-18, 20, 23-28, 30,31, 33,36 |
| | ----- | |

Form PCT/ISA/210 (continuation of second sheet) (April 2005)

## INTERNATIONAL SEARCH REPORT

Information on patent family members

International application No
PCT/CU2021/050012

| Patent document cited in search report | Publication date | Patent family member(s) | Publication date |
|---|---|---|---|
| US 2016106691 A1 | 21-04-2016 | CA 2904762 A1 | 04-09-2014 |
| | | CU 20130027 A7 | 30-10-2014 |
| | | JP 6595345 B2 | 23-10-2019 |
| | | JP 2016510007 A | 04-04-2016 |
| | | US 2016106691 A1 | 21-04-2016 |
| | | WO 2014131374 A1 | 04-09-2014 |
| | | ZA 201506583 B | 31-07-2019 |
| WO 2020094161 A1 | 14-05-2020 | CA 3118959 A1 | 14-05-2020 |
| | | CN 113226381 A | 06-08-2021 |
| | | CU 20180138 A7 | 20-10-2020 |
| | | EP 3878475 A1 | 15-09-2021 |
| | | JP 2022515706 A | 22-02-2022 |
| | | KR 20210091216 A | 21-07-2021 |
| | | US 2021290782 A1 | 23-09-2021 |
| | | WO 2020094161 A1 | 14-05-2020 |
| EP 3878475 A1 | 15-09-2021 | CA 3118959 A1 | 14-05-2020 |
| | | CN 113226381 A | 06-08-2021 |
| | | CU 20180138 A7 | 20-10-2020 |
| | | EP 3878475 A1 | 15-09-2021 |
| | | JP 2022515706 A | 22-02-2022 |
| | | KR 20210091216 A | 21-07-2021 |
| | | US 2021290782 A1 | 23-09-2021 |
| | | WO 2020094161 A1 | 14-05-2020 |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5382601 A
- US 20060051416 A
- WO 2010118706 A4, Carrazana
- WO 2014131374 A1
- US 20030225031 A1, Quay, S.C.
- US 20060003989 A1
- US 20050245617 A1, Went G.T.
- US 20060252788 A1
- US 20060189694 A1
- US 20070037800 A1, Cummings, C.J.,
- CN 1621039, Tao, T.


### Non-patent literature cited in the description


- **HARDY JA et al.** *Science,* 1992, vol. 256, 184-5
- **HAASS C. et al.** *Cold Spring Harb Perspect Med.,* 2012, vol. 2, a006270
- **MUCKE L et al.** *Cold Spring Harb Perspect Med.,* 2012, vol. 2, a006338
- **CASTELLO MA et al.** *Aging Res Rev.,* 2013, vol. 12, 282-8
- **CASTELLO MA et al.** *Aging Res Rev.,* 2014, vol. 13, 10-2
- **DRACHMAN DA.** *Alzheimers Dement,* 2014, vol. 10, 372-80
- **FERREIRA ST et al.** *Alzheimers Dement,* 2014, vol. 10 (1), S76-83
- **DE FELICE FG et al.** *Diabetes,* 2014, vol. 63, 2262-72
- **DE FELICE FG.** *J Clin Invest.,* 2013, vol. 123, 531-9
- **COCHRAN JN et al.** *Brain Res Bull.,* 2014, vol. 103, 18-28
- **REITZ C.** *International Journal of Alzheimer's disease.,* January 2012, vol. 2012, 369808
- **RODRIGUE K.** *Neuropsychol Rev.,* December 2009, vol. 19 (4), 436-450
- **BATEMAN, RJ et al.** Alzheimer's disease. *N. Engl. J Med.,* 2012, vol. 367, 795-804
- **KARRAN, E. et al.** *Nat. Rev. Drug Discov.,* 2011, vol. 10, 698-712
- **SOYON HONG et al.** *Science,* 2016
- **MALM TM.** *Neurotherapeutics,* 2015, vol. 12, 81-93
- **ELALI A. et al.** *Brain, Behavior, and Immunity,* 2015
- **MICHAUD J.-P. et al.** *Neuron,* 2015, vol. 85 (4), 450-2
- **MAURER, I et al.** *Neurobiol. Aging,* 2000, vol. 21, 455-462
- **CASPERSEN, C. et al.** *FASEB J.,* 2005, vol. 19, 2040-2041
- **MOSCONI, L et al.** *Ann. NY Acad. Sci.,* 2008, vol. 1147, 180-195
- **LUSTBADER et al.** *Science,* 2004, vol. 304, 448-0452
- **MANCZAK et al.** *Hum. Mol. Genet.,* 2006, vol. 15, 1437-1449
- **YATES et al.** *J. Neurochem,* 1990, vol. 55, 1624-1630
- **MAURER, I. et al.** *Neurobiol Aging,* 2000, vol. 21, 455-462
- **LEUNER et al.** *Mol. Neurobiol.,* 2012, vol. 46, 186-193
- **CASLEY et al.** *Neurobiol. Dis.,* 2002, vol. 10, 258-267
- **ALEARDI, AM et al.** *J. Bioenerg. Biomembr.,* 2005, vol. 37, 207-225
- **CLEMENTI, ME et al.** *FEBS Lett.,* 2005, vol. 579, 2913-2918
- **SMITH, MA et al.** *Nature,* 1996, vol. 382, 120-121
- **KROEMER, G. ; REED, JC.** *Nat. Med.,* 2000, vol. 6, 513-519
- **JÜRGENSMEIER, JM et al.** *Proc. Natl. Acad. Sci. USA,* 1998, vol. 95, 4997-5002
- **MARCH, I. et al.** *Science,* 1998, vol. 281, 2027-2031
- **NARITA, M. et al.** *Proc. Natl. Acad. Sci. USA,* 1998, vol. 95, 14681-14686
- **LIU, X. et al.** *Cell,* 1996, vol. 86, 147-157
- **YANG, J et al.** *Science,* 1997, vol. 275, 1129-1132
- **ZÁDORI D.** *Journal of Alzheimer's Disease,* 2018, vol. 62, 523-547
- **VILLEGAS S.** *Medicina Clinica,* 2015, vol. 145 (2), 76-83
- **CHIANG K et al.** *Annu Rev Pharmacol Toxicol.,* 2014, vol. 54, 381-405
- **FRANCIS PT et al.** *Trends Pharmacol Sci.,* 2005, vol. 26, 104-11

- **HUANG Y. et al.** *Cell,* 2012, vol. 148, 1204-22
- **THOMSON PDR ET AL.** *Physicians Desk Reference,* 2008, vol. 2011 (19), 1
- **THOMSON PDR et al.** *Clinical Geriatrics,* vol. 2011 (19), 1
- **ALI T. et al.** *PLOS ONE,* 2015, vol. 7, 1-10
- **TSOI KKF.** *JAMDA,* 2015, vol. 1-7
- **O'BRIEN JT, et al.** *J Psychopharmacol Oxf Engl.,* 2011, vol. 25, 997-1019
- **HUANG Y et al.** *Cell,* 2012, vol. 148, 1204-22
- **ZAWIEJA P et al.** *Geriatr Gerontol. Int.,* 2012, vol. 12, 365-71
- **FROLICH L et al.** *J Alzheimers Dis.,* 2011, vol. 24, 363-74
- **AISEN PS et al.** *Arch Med Sci.,* 2011, vol. 7, 102-11
- **CALTAGIRONE C et al.** *Alzheimers Dis.,* 2010, vol. 20, 509-16
- **SALLOWAY S et al.** *Neurology.,* 2011, vol. 77, 1253-62
- **YAN R et al.** *Lancet Neurol.,* 2014, vol. 13, 319-29
- **VELLAS B et al.** *Curr Alzheimer Res.,* 2011, vol. 8, 203-12
- **XIA W.** *J Alzheimers Dis.,* 2012, vol. 31, 685-96
- *Drug Saf.,* 2013, vol. 22, 345-58
- **PANZA F. et al.** Active Immunotherapy. *Expert Rev Clin Immunol.,* 2014, vol. 10, 405-19
- **RYAN R. JM et al.** *J Alzheimers Dis.,* 2009, vol. 17, 243
- **WINBLAD B. et al.** *Lancet Neurol.,* 2012, vol. 11, 597-604
- **SCHNEEBERGER A. et al.** *N Engl J Med.,* 2014, vol. 370, 322-33
- **PANZA F. et al.** *Expert Rev Clin Immunol.,* 2014, vol. 10, 405-19
- **SALLOWAY S. et al.** *N Engl J Med.,* 2014, vol. 370, 322-33
- **DOODY RS. et al.** *N. Engl J Med.,* 2014, vol. 370, 311-21
- **OSTROWITZKI S. et al.** *in Arch Neurol.,* 2012, vol. 69, 198-207
- **RODRIGUEZ, IC ; CEREZO, A.** Salem, in II Bioadhesive delivery systems. *Ars Pharmaceutica,* 2000, vol. 41 (1), 115-128
- **VINTILOIU, A. ; LEROUX, JC.** Organogels and their use in drug delivery. *Journal of Controlled Release,* 2008, vol. 125, 179-192
- **FRENKEL, J.** *Rubber Chemistry and Technology,* 1940, vol. 13, 264-274
- **TANAKA et al.** Encyclopedia of Polymer Science. 1986, vol. 6, 514
- **FYFE, CA ; BLAZER, AI.** *Journal of Controlled Release,* 1998, vol. 52, 221-225
- **DABBAGH, MA et al.** *International. Journal of Pharmaceutics,* 1996, vol. 140, 85-95
- **MICHAEL IU et al.** *J Pharm Pharmacol,* 2001, vol. 53, 3-22
- **ILIUM L.** *Eur J Pharm Sci,* 2000, vol. 11, 1-18
- **FREY WH II.** *Drug Deliv Tech,* 2002, vol. 2, 46-49
- **VYAS TK et al.** *Curr Drug Deliv,* 2005, vol. 2 (2), 164-175
- **THORNE RG et al.** *Neuroscience,* 2004, vol. 127, 481-496
- **PIRES, A et al.** *J Pharm. Pharmaceut Sci.,* 2009, vol. 12 (3), 288-311
- **PIRES, A. et al.** *J Pharm. Pharmaceut Sci.,* 2009, vol. 12 (3), 288-311
- **CASETTARI, L. ; ILLUM, L.** *J. Control. Release,* 2014, vol. 190, 189-200
- **MANSURI, S. et al.** *React. Funct. Polym.,* 2016, vol. 100, 481151-172
- **BEHL CR et al.** *Adv Drug Del Rev,* 1998, vol. 29 (1), 89-116
- **BAJO R et al.** *Scientific reports,* 2015, vol. 5, 9748
- **GILLES C ; ERTLE S.** *Dialogues Clin. Neurosci.,* 2000, vol. 2 (3), 247-255
- **HAIDER S et al.** *in Brain Res. Bull.,* 2016, vol. 127, 234-247
- **SHIN CY et al.** *Biomolecules & Therapeutics,* 2018, vol. 26 (3), 274-281
- **HAIDER et al.** *Brain Research Bulletin,* 2016, vol. 127, 234-247
- **JUNG et al.** *Biol. Pharm. Bull.,* 2009, vol. 32 (2), 242-246
- **KONAR et al.** *PLOS ONE,* 2011, vol. 6 (11), e27265
- **LEE et al.** *Scientific reports,* 2015, vol. 5, 9651
- **WONG-GUERRA et al.** *Neurol Res.,* 2017, vol. 39 (7), 649-659
- **MCGAUGH JL.** *Science,* 1966, vol. 153, 1351
- **CSISZAR A. et al.** *Am. J. Physiol. Heart Circ. Physiol.,* 2013, vol. 305, H1120-1130
- **OLTON DS ; PARAS BC,.** *Neuropsychologia,* 1979, vol. 17, 669-682
- **LEE JS et al.** *Scientific reports,* 2015, vol. 5, 9651
- **RIEDEL G et al.** *Behav. Brain Res.,* 2009, vol. 204 (1), 217-225
- **MORRIS R,.** Morris's Water Maze. *Journal of Neuroscience Methods,* 1984, vol. 11 (1), 47-6
- **GULINELLO M. ; M. GERTNER et al.** *Behav. Brain Res.,* 2009, vol. 196, 220-227
- **KLINKENBERG I ; BLOKLAND A,.** *Neurosci Biobehav Rev,* 2010, vol. 34 (8), 1307-1350
- **HAIDER, S. et al.** *Brain research bulletin,* 2016, vol. 127, 234-247
- **BIHAQI SW, et al.** *Indian J. Pharmacol.,* 2012, vol. 44 (5), 593-598
- **RAMANDEEP K et al.** *Int J Prev Med Res.,* 2015, vol. 1 (2), 45-64

- **WANG Q et al.** *Mol Neurobiol.,* 2005, vol. 31, 3-16
- **CHEN, Z. et al.** *J. Neurobiol.,* 2005, vol. 62 (2), 207-218
- **BRORSON, JR et al.** *J. Neurosci.,* 1994, vol. 14 (1), 187-197
- **WANG, Q. et al.** *Mol. Neurobiol.,* 2005, vol. 31 (1-3), 3-16
- **UEDA, Y. et al.** *Exp. Brain Res.,* 2002, vol. 147 (2), 219-226
- **THORNE, RG et al.** *Neuroscience,* 2002, vol. 127, 481-496
- **DE BRUIN N, et al.** *J Neurodegener Dis.,* 2015, vol. 2015, 242505
- **MIRANDOLA SR et al.** *J Neurosci Res,* 2010, vol. 88, 630-39
- **ZANOTTI A. ; AZZONE GF.** *Archives of Biochemistry and Biophysics,* 1980, vol. 201 (1), 255-265
- **AMPLEX RED.** *Molecular Probes, OR, Eugene*
- **VOTYAKOVA ; REYNOLDS.** *J Neurochem.,* 2001, vol. 79 (2), 266-77
- **MIRANDA HF et al.** *J. Biomed. Sci.,* 2014, vol. 21 (1), 62-62
- **ELLMAN GL et al.** *Biochem Pharmacol.,* 1961, vol. 7, 88-95
- **MARKWELL MAK et al.** *Anal. Biochem.,* 1978, vol. 87 (1), 206-210
- **PARK JH, et al.** *In J Mol Neurosci.,* 2016, vol. 59 (4), 579-589
- **R.J. RACINE.** *Clin. Neurophysiol.,* 1972, vol. 32, 281-294
- USP. Mack Printing, 2013, vol. 36, 3220-3221
- **HANSON, L.R. et al.** *inJ. Vis. Exp.,* 2013, vol. 74, e4440